# EUROPEAN PATENT SPECIFICATION

(11) **EP 3 392 269 B1**
(45) Date of publication and mention of the grant of the patent: **27.10.2021**
(21) Application number: 18170211.9
(22) Date of filing: 15.03.2010
(51) Int. Cl.: A61P 3/10, A61P 1/04, A61P 9/10, C07K 14/47, C07K 14/78, C07K 16/18, A61P 11/00, A61P 11/06, A61P 17/02, A61P 17/06, A61P 19/02, A61P 25/00, A61P 29/00, A61P 31/00, A61P 35/00, A61P 37/00, A61P 43/00

(54) **ANTI TENASCIN C ANTIBODIES TO TREAT INFLAMMATORY DISEASES**
ANTI-TENASCIN-C-ANTIKÖRPER ZUR BEHANDLUNG ENTZÜNDLICHER ERKRANKUNGEN
ANTICORPS ANTI-TENASCIN C POUR LE TRAITEMENT DES MALADIES INFLAMMATOIRES

(30) Priority: 13.03.2009 GB 0904355
(43) Date of publication of application: 24.10.2018
(62) Divisional of application: 14162944.4
(73) Proprietor: Imperial Innovations Limited, London SW7 2PG (GB)
(72) Inventor: Midwood, Kim Suzanne, Headington, Oxford OX3 7FY (GB); Foxwell, Brian Maurice John, deceased (GB)
(74) Representative: Sterling IP

(56) References cited:
- US-A- 6 124 260
- STEFAN RIEDL ET AL: "Serum tenascin-C is an indicator of inflammatory bowel disease activity", INTERNATIONAL JOURNAL OF COLORECTAL DISEASE, vol. 16, no. 5, 13 June 2001 (2001-06-13), pages 285-291, XP055506936, DE ISSN: 0179-1958, DOI: 10.1007/s003840100312
- CHIQUET-EHRISMANN RUTH ET AL: "Tenascins: regulation and putative functions during pathological stress.", THE JOURNAL OF PATHOLOGY JUL 2003 LNKD- PUBMED:12845616, vol. 200, no. 4, July 2003 (2003-07), pages 488-499, XP002594134, ISSN: 0022-3417
- Kim Midwood ET AL: "Tenascin-C is an endogenous activator of Toll-like receptor 4 that is essential for maintaining inflammation in arthritic joint disease", Nature Medicine, vol. 15, no. 7, 28 June 2009 (2009-06-28), pages 774-780, XP055532191, New York ISSN: 1078-8956, DOI: 10.1038/nm.1987

## Description

The present disclosure relates to tenascin-C and its activity in chronic inflammation. There is also provided modulators of tenascin-C and its biological activity and further uses of tenascin-C in the identification of agents that up-regulate or down-regulate chronic inflammation.

Inflammation is the complex biological response of tissues to harmful stimuli, such as pathogens, tissue damage, or irritants. It is a protective attempt by the tissue to remove the injurious stimuli as well as initiate the healing process for the tissue. Abnormalities associated with inflammation comprise a large, unrelated group of disorders which underlie a variety of human diseases (inflammatory disorders). Examples of diseases with an inflammatory aspect include (but are not limited to) asthma, autoimmune disease, glomerulonephritis, allergy (hypersensitivities), inflammatory bowel diseases, reperfusion injury, rheumatoid arthritis and transplant rejection.

In particular, chronic inflammation is a debilitating and serious condition associated with many of the above diseases and is characterised by persistent inflammation at a site of infection or injury, or in relation to altered immune responses such as in autoimmune disease.

Rheumatoid arthritis (RA) is a typical example of a chronic inflammatory condition. RA is characterized by synovial inflammation and destruction of joint cartilage and bone mediated by persistent synthesis of pro-inflammatory cytokines and matrix metalloproteinases (MMPs). Biological compounds that suppress the synthesis of inflammatory cytokines such as TNFα and IL-6 are successful at treating RA in the short-term. However, repeated treatments are required, which renders this an expensive therapeutic approach, and does not provide long-term remission. Furthermore, total systemic suppression of cytokine function is not without inherent problems, such as increased infectious risk. Thus, despite advances in care, there remains an unmet need for an economical mode of treatment of chronic inflammatory that is efficacious over the long term (Smolen (2006) and Williams (2007)).

The mechanisms that underpin disease chronicity remain unclear and the factor(s) that drive the prolonged expression of inflammatory and destructive mediators are currently unknown.

Toll-like receptors (TLRs) play a key role in driving the production of inflammatory mediators in RA and blockade of TLR function may be of significant clinical benefit (reviewed in Brentano (2005) and O'Neill (2002)). This receptor family forms an integral part of the immune system. TLRs mediate host defence against infection and injury by recognising both pathogen-associated molecular patterns (PAMPs) and damage-associated molecular patterns (DAMPs) (Matzinger (2002)). DAMPs are endogenous pro-inflammatory molecules generated upon tissue injury and include intracellular molecules released from damaged or necrotic cells, fragments of extracellular matrix (ECM) molecules or ECM molecules up regulated upon injury (reviewed in Bianchi (2007) and Gordon (2002)).

Upon activation, TLRs promote both innate and adaptive immune responses including stimulation of expression of pro-inflammatory cytokines and MMPs (Medzhitov (2002)). TLRs are expressed at high levels in synovial tissue from RA patients (Radstake (2004), Roelofs (2005), Sacre (2007), and (Sacre, manuscript submitted 2008) and mice with targeted deletions or loss of function mutations in TLR4 are protected from experimental arthritis (Choe (2003) and Lee (2005). Furthermore, inhibitors of TLR4 can reduce destructive arthritis in mice (Abdollahi-Roodsaz (2007)) and a putative TLR4 inhibitor improved symptoms in 15 out of 23 patients with moderate to severe RA in a preliminary phase I trial (Vanags (2006). However, it is unclear which TLR ligand(s) are involved in disease pathogenesis.

US6,124,260 describes the inhibition of smooth muscle cell migration by tenascin-C peptides.

Tenascin-C is an ECM glycoprotein that is associated with tissue injury and wound repair. Tenascin-C is expressed specifically during active tissue remodelling during embryogenesis, being first observed during gastrulation and somite formation. In later stages of development expression is restricted to sites of branching morphogenesis of mammary gland and the lung, in the developing skeleton, cardiovascular system and in connective tissues at sites of epithelial to mesenchymal transformation. Expression is down regulated once these processes cease and before embryogenesis is complete (Jones (2000)).

Tenascin-C is not normally expressed in healthy adult tissue but, in adults, is specifically and transiently up-regulated during acute inflammation and persistently expressed in chronic inflammation (reviewed in Chiquet-Ehrismann (2003)). Immunohistochemical studies show that little tenascin-C is expressed in normal human joints but levels are greatly increased in RA synovia, in areas of inflammation and fibrosis, specifically below the synovial lining, in the invading pannus and around blood vessels (Cutolo (1992), MacCachren (1992) and Salter (1993)). There is also a significant increase in tenascin-C levels in synovial fluid from RA patients (Chevalier (1994) and Hasegawa (2007)) and in RA cartilage (Salter (1993) and Chevalier (1994)).

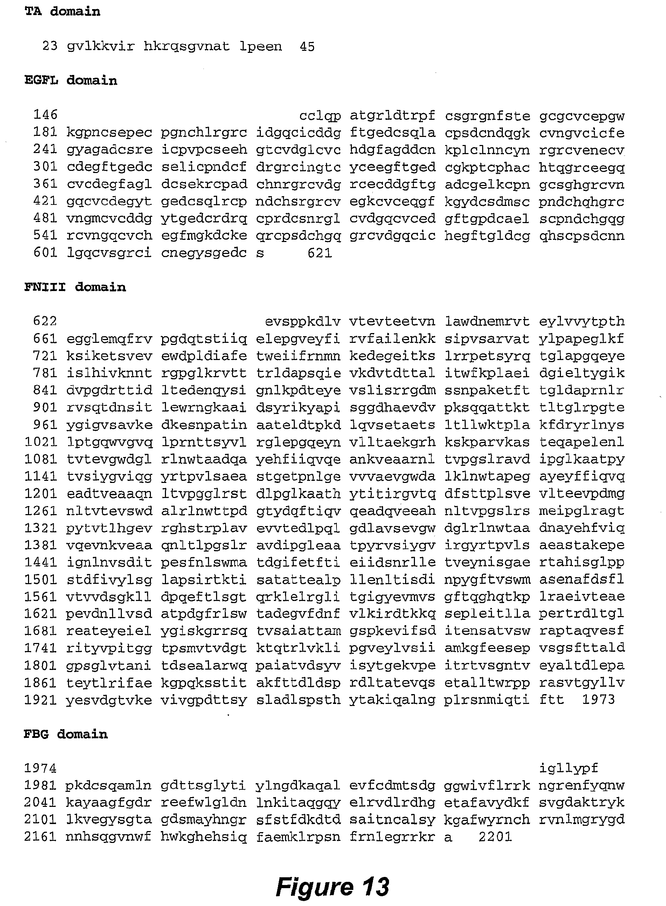

Tenascin-C is a large hexameric protein of 1.5 million Da. Each chain comprises different domains, including an assembly domain (TA), EGF-like repeats (EGF-L), fibronectin type III-like repeats (TNIII) and a fibrinogen-like globe (FBG) (reviewed in Orend (2005)). The sequences of tenascin-C and its domains are shown in Figure 13.

Previously, the role of tenascin-C in inflammation has been uncertain, with evidence showing varying effects on different immune cells. For example tenascin-C has been shown to supports primary human peripheral blood and tonsillar lymphocyte adhesion and rolling, thereby suggesting a role in stimulating lymphocyte migration (Clark (1997)). In addition, tenascin-C null mice exhibit reduced lymphocyte infiltration and lower levels of IFN, TNF and IL-4 mRNA upon concanavalin A-induced liver injury in mice (El-Karef (2007)). Thus, evidence suggests tenascin-C is involved in promoting activity of acute inflammatory cells. However, tenascin-C has also been reported to inhibit monocyte chemotaxis *in vitro* (Loike (2001)) and tenascin-C-null mice exhibit increased migration of monocytes and macrophages in mammary tumour stroma (Talts (1999)). This evidence therefore suggests tenascin-C has a role in inhibition of inflammatory cells.

The inventors have shown that tenascin-C is an endogenous TLR4 ligand that it is required for destructive joint inflammation observed in arthritis.

Furthermore, it is now shown that tenascin-C is not involved with induction of inflammation (acute inflammatory response) but instead is involved in the prolonging of the inflammatory response characterising the chronic inflammatory condition. In particular, tenascin-C has now been shown to be an endogenous activator of TLR4 and demonstrated that this molecule is required for destructive joint inflammation.

A role for tenascin-C in mediating an immune response in the joint was demonstrated by induction of joint inflammation upon intra-articular injection of the FBG domain of tenascin-C in mice *in vivo.* Moreover, acute joint inflammation induced by zymosan was not as prolonged in tenascin-C deficient mice. Both the wild type and tenascin-C null mice responded to acute inflammation induction by zymosan equally, demonstrating that tenascin-C does not appear to be involved in the initiation of inflammation. However, the less persistent synovitis exhibited by tenascin-C null mice indicates a role in the maintenance of joint inflammation. The importance of tenascin-C in prolonging joint inflammation was underscored by the observation that targeted deletion of tenascin-C protected mice from sustained erosive joint inflammation during arthritis induced by immunization with mBSA.

Tenascin-C has now been shown to be capable of activating cells in the joint and the primary active domain of tenascin-C has been mapped to the fibrinogen-like globe (FBG), a 227 amino acid (26.9 kDa) globular domain at the C terminal of the molecule (Siri (1991)).

Addition of FBG to synovial membrane cultures from RA patients enhanced the spontaneous release of pro-inflammatory cytokines. It also stimulated synthesis of TNF-α, IL-6 and IL-8 in primary human macrophages and IL-6 in RA synovial fibroblasts via activation of TLR4 and MyD88 dependent signalling pathways.

It has now been shown that, as in the case of LPS, TLR4 expression is necessary for induction of cytokine synthesis by FBG. However, unlike LPS, neither CD14 nor MD-2 appears to be required for TLR-4 activation. CD14 is dispensable for activation of TLR4 by other ligands. It is not required for TLR4 to respond to lipid A in a MyD88 dependent manner (Jiang (2005)), fibronectin EDA can activate mast cells even in the absence of CD14 (Gondokaryono (2007)) and hyaluronic acid activation of human monocytic THP-1 cells requires a complex of TLR4, CD44 and MD-2, but not CD14 (Taylor (2007)).

Formation of distinct receptor complexes by each TLR4 ligand may facilitate recruitment of different intracellular adapter/signalling molecules. This may account for the differential cellular responses we observe with FBG and LPS, for example lack of IL-8 induction by FBG in RA synovial fibroblasts. Similarly, hyaluronic acid activation of the TLR4 and CD44 complex induces a pattern of gene expression in mouse alveolar macrophage cell lines that is different to LPS (Taylor (2007)). That FBG induces IL-8 synthesis in human macrophages, suggests cell type specific ligand recognition and/or signalling occurs.

The tightly regulated pattern of expression of tenascin-C makes it an attractive target for treating chronic inflammation. It is predominantly absent from healthy adults, however expression is specifically induced upon tissue injury. During acute inflammation tenascin-C is transiently expressed: induction often precedes inflammation and both mRNA and protein are absent from the tissue by the time inflammation is resolved (reviewed in Chiquet-Ehrismann (2003)).

Persistent expression of tenascin-C has now been shown to be associated with chronic inflammation. In addition to RA, increased tenascin-C levels are observed in other autoimmune diseases including multiple sclerosis (Gutowski (1999)) and Sjogrens disease (Amin (2001)), and in non-healing wounds and diabetic and venous ulcers (Loots (1998)). *De novo* synthesis of tenascin-C correlates well with the intensity of inflammation in diseases of the oral mucosa and plasma levels of tenascin-C are a reliable indicator for the activity of inflammatory bowel diseases before and after medication or surgery (reviewed in Chiquet-Ehrismann (2003)).

In a first aspect of the invention there is provided an agent for use in the treatment of chronic inflammations selected from rheumatoid arthritis and Sjogren's disease, wherein the agent modulates the biological activity of tenascin-C by inhibiting the binding properties of Tenascin C, and the agent is an antibody or antigen-binding fragment thereof having specificity to FBG domain that activates TLR4, wherein said antibody or antigen-binding fragment thereof specifically binds to at least one peptide selected from:
- Peptide 5 ELRVDLRDHGETAFAVYDKFSVGDAKTRYK
- Peptide 6 KTRYKLKVEGYSGTAGDSMAYHNGRSFST, and
- Peptide 7 RSFSTFDKDTDSAITNCALSYKGAFWYRN.

Such agents may be identified using methods well known in the art, such as:
(a) by determining the effect of a test agent on levels of expression of tenascin-C, for example by Southern blotting or related hybridisation techniques;
(b) by determining the effect of a test agent on levels of tenascin-C protein, for example by immunoassays using anti- tenascin-C antibodies; and
(c) by determining the effect of a test agent on a functional marker or result of tenascin-C activity, for example via the methods of the examples.

The agent for use in the first aspect of the invention may down-regulate the biological activity of tenascin-C.

The agent for use the first aspect of the invention may be an inhibitor of the binding properties of tenascin-C. For example, the agent may alter the conformation of tenascin-C such that it is no longer able to bind to its receptor.

The agent for use the first aspect of the invention may be a competitive binding inhibitor of tenascin-C.

The agent for use in the first aspect of the invention may be an antagonist of the TLR-4 receptor.

It will be appreciated by persons skilled in the art that inhibition of the biological activity of tenascin-C by an agent of the disclosure may be in whole or in part. For example, the agent may inhibit the biological activity of tenascin-C by at least 10%, preferably at least 20%, 30%, 40%, 50%, 60%, 70%, 80% or 90%, and most preferably by 100% compared to the biological activity of tenascin-C on inflammatory cells which have not been exposed to the agent.

The agent for use in the first aspect of the invention may be selected from the group consisting of antibodies (polyclonal or monoclonal) and antigen-binding fragments thereof.

The FBG domain has been shown to be predominantly involved in the interaction of tenascin-C with its target in relation to the persistence of chronic inflammation (sequence shown in figure 13).

A compound with binding affinity for tenascin-C may bind substantially reversibly or substantially irreversibly to an active site of tenascin-C. The compound may bind to a portion of tenascin-C that is not the active site so as to interfere with the binding of the tenascin-C to a ligand or receptor. The compound may bind to a portion of tenascin-C so as to decrease the proteins activity by an allosteric effect. This allosteric effect may be an allosteric effect that is involved in the natural regulation of the activity of Tenascin-C, for example in the activation of the tenascin-C by an "upstream activator".

Methods for detecting interactions between a test compound and tenascin-C are well known in the art. For example, ultrafiltration with ion spray mass spectroscopy/HPLC methods or other physical and analytical methods may be used. In addition, Fluorescence Energy Resonance Transfer (FRET) methods may be used, in which binding of two fluorescent labelled entities may be measured by measuring the interaction of the fluorescent labels when in close proximity to each other.

Alternative methods of detecting binding of a polypeptide to macromolecules, for example DNA, RNA, proteins and phospholipids, include a surface plasmon resonance assay, for example as described in Plant et al., 1995, Analyt Biochem 226(2), 342-348. Methods may make use of a polypeptide that is labelled, for example with a radioactive or fluorescent label.

A further method of identifying a compound that is capable of binding to the polypeptide is one where the polypeptide is exposed to the compound and any binding of the compound to the said polypeptide is detected and/or measured. The binding constant for the binding of the compound to the polypeptide may be determined. Suitable methods for detecting and/or measuring (quantifying) the binding of a compound to a polypeptide are well known to those skilled in the art and may be performed, for example, using a method capable of high throughput operation, for example a chip-based method. New technology, called VLSIPS™, has enabled the production of extremely small chips that contain hundreds of thousands or more of different molecular probes. These biological chips or arrays have probes arranged in arrays, each probe assigned a specific location. Biological chips have been produced in which each location has a scale of, for example, ten microns. The chips can be used to determine whether target molecules interact with any of the probes on the chip. After exposing the array to target molecules under selected test conditions, scanning devices can examine each location in the array and determine whether a target molecule has interacted with the probe at that location.

Another method of identifying compounds with binding affinity for tenascin-C is the yeast two-hybrid system, where the polypeptides of the disclosure can be used to "capture" proteins that bind tenascin-C. The yeast two-hybrid system is described in Fields & Song, Nature 340:245-246 (1989).

The agent for use in the first aspect of the invention is an antibody or antigen-binding fragment thereof. The antigen-binding fragment may be selected from the group consisting of Fv fragments (e.g. single chain Fv and disulphide-bonded Fv), Fab-like fragments *(e.g*. Fab fragments, Fab' fragments and F(ab)₂ fragments), single variable domains (e.g. V_{H} and V_{L} domains) and domain antibodies (dAbs, including single and dual formats [*i.e.* dAb-linker-dAb]).

The antibody may preferably bind specifically to the FBG domain that activates TLR4.

The advantages of using antibody fragments, rather than whole antibodies, are several-fold. The smaller size of the fragments may lead to improved pharmacological properties, such as better penetration of solid tissue. Moreover, antigen-binding fragments such as Fab, Fv, ScFv and dAb antibody fragments can be expressed in and secreted from *E. coli,* thus allowing the facile production of large amounts of the said fragments.

Also included within the scope of the invention are modified versions of antibodies and an antigen-binding fragments thereof, e.g. modified by the covalent attachment of polyethylene glycol or other suitable polymer.

Methods of generating antibodies and antibody fragments are well known in the art. For example, antibodies may be generated via any one of several methods which employ induction of *in vivo* production of antibody molecules, screening of immunoglobulin libraries (Orlandi. et al, 1989. Proc. Natl. Acad. Sci. U.S.A. 86:3833-3837; Winter et al., 1991, Nature 349:293-299) or generation of monoclonal antibody molecules by cell lines in culture. These include, but are not limited to, the hybridoma technique, the human B-cell hybridoma technique, and the Epstein-Barr virus (EBV)-hybridoma technique (Kohler et al., 1975. Nature 256:4950497; Kozbor et al., 1985. J. Immunol. Methods 81:31-42; Cote et al., 1983. Proc. Natl. Acad. Sci. USA 80:2026-2030; Cole et al., 1984. Mol. Cell. Biol. 62:109-120).

Suitable monoclonal antibodies to selected antigens may be prepared by known techniques, for example those disclosed in *"*Monoclonal Antibodies: A manual of techniques", H Zola (CRC Press, 1988) and in *"*Monoclonal Hybridoma Antibodies: Techniques and Applications", J G R Hurrell (CRC Press, 1982).

Antibody fragments can be obtained using methods well known in the art (see, for example, Harlow & Lane, 1988, "Antibodies: A Laboratory Manual", Cold Spring Harbor Laboratory, New York). For example, antibody fragments employed in the present invention can be prepared by proteolytic hydrolysis of the antibody or by expression in *E. coli* or mammalian cells (*e.g.* Chinese hamster ovary cell culture or other protein expression systems) of DNA encoding the fragment. Alternatively, antibody fragments can be obtained by pepsin or papain digestion of whole antibodies by conventional methods.

It will be appreciated by persons skilled in the art that for human therapy or diagnostics, humanised antibodies are preferably used. Humanised forms of non-human (e.g. murine) antibodies are genetically engineered chimaeric antibodies or antibody fragments having preferably minimal-portions derived from non-human antibodies. Humanised antibodies include antibodies in which complementary determining regions of a human antibody (recipient antibody) are replaced by residues from a complementary determining region of a non human species (donor antibody) such as mouse, rat of rabbit having the desired functionality. In some instances, Fv framework residues of the human antibody are replaced by corresponding non-human residues. Humanised antibodies may also comprise residues which are found neither in the recipient antibody nor in the imported complementarity determining region or framework sequences. In general, the humanised antibody will comprise substantially all of at least one, and typically two, variable domains, in which all or substantially all of the complementarity determining regions correspond to those of a non human antibody and all, or substantially all, of the framework regions correspond to those of a relevant human consensus sequence. Humanised antibodies optimally also include at least a portion of an antibody constant region, such as an Fc region, typically derived from a human antibody (see, for example, Jones et al., 1986. Nature 321:522-525; Riechmann et al., 1988, Nature 332:323-329; Presta, 1992, Curr. Op. Struct. Biol. 2:593-596).

Methods for humanising non-human antibodies are well known in the art. Generally, the humanised antibody has one or more amino acid residues introduced into it from a source which is non-human. These non-human amino acid residues, often referred to as imported residues, are typically taken from an imported variable domain. Humanisation can be essentially performed as described (see, for example, Jones et al., 1986, Nature 321:522-525; Reichmann et al., 1988. Nature 332:323-327; Verhoeyen et al., 1988, Science 239:1534-15361; US 4,816,567) by substituting human complementarity determining regions with corresponding rodent complementarity determining regions. Accordingly, such humanised antibodies are chimaeric antibodies, wherein substantially less than an intact human variable domain has been substituted by the corresponding sequence from a non-human species. In practice, humanised antibodies may be typically human antibodies in which some complementarity determining region residues and possibly some framework residues are substituted by residues from analogous sites in rodent antibodies.

Human antibodies can also be identified using various techniques known in the art, including phage display libraries (see, for example, Hoogenboom & Winter, 1991, J. Mol. Biol. 227:381; Marks et al., 1991, J. Mol. Biol. 222:581; Cole et al., 1985, In: Monoclonal antibodies and Cancer Therapy, Alan R. Liss, pp. 77; Boerner et al., 1991. J. Immunol. 147:86-95).

Once suitable antibodies are obtained, they may be tested for activity, for example by ELISA.

Co-receptors to primary receptors, such as TLR4, assist with binding of a signalling molecule to the primary receptor in order to facilitate ligand recognition and binding and initiate/maintain the biological process resulting from receptor binding.

The agent employed in the first aspect of the invention may be an antibody or antigen-binding fragment thereof has specificity for the FBG domain of tenascin-C.

The method of identifying an agent that modulates the activity of tenascin-Cmay employ one or more cells selected from the group consisting of inflammatory cells, fibroblasts, fibroblast like cells (including RA synovial fibroblasts, also known as synoviocytes), mouse embryonic fibroblasts, human embryonic kidney cells.

The inflammatory cells may be selected from the group consisting of macrophages, dendritic cells, monocytes, lymphocytes, monocyte like cells and macrophage like cells. identifying an agent described above.

Chronic inflammation may be associated with any condition associated with inappropriate inflammation. Such conditions include, but are not limited to, rheumatoid arthritis (RA), autoimmune conditions, inflammatory bowel diseases, non-healing wounds, multiple sclerosis, cancer, atherosclerosis, sjogrens disease, diabetes, lupus erythrematosus (including systemic lupus erythrematosus), asthma, fibrotic diseases (including liver cirrhosis), pulmonary fibrosis, UV damage and psoriasis.

Of particular, but non-exclusive interest, the chronic inflammation is associated with rheumatoid arthritis (RA).

In a further aspect of the disclosure there is provided a composition comprising an agent according to the present disclosure and a pharmaceutically acceptable carrier, excipient and/or diluent.

It will be appreciated by persons skilled in the art that such an effective amount of the agent or formulation thereof may be delivered as a single bolus dose (*i.e.* acute administration) or, more preferably, as a series of doses over time (*i.e.* chronic administration).

The agents of the disclosure can be formulated at various concentrations, depending on the efficacy/toxicity of the compound being used and the indication for which it is being used. Preferably, the formulation comprises the agent of the invention at a concentration of between 0.1 µM and 1 mM, more preferably between 1 µM and 100 µM, between 5 µM and 50 µM, between 10 µM and 50 µM, between 20 µM and 40 µM and most preferably about 30 µM. For *in vitro* applications, formulations may comprise a lower concentration of a compound of the invention, for example between 0.0025 µM and 1 µM.

It will be appreciated by persons skilled in the art that the agents for use in the invention will generally be administered in admixture with a suitable pharmaceutical excipient diluent or carrier selected with regard to the intended route of administration and standard pharmaceutical practice (for example, see Remington: The Science and Practice of Pharmacy, 19th edition, 1995, Ed. Alfonso Gennaro, Mack Publishing Company, Pennsylvania, USA).

For example, the agents of the disclosure can be administered orally, buccally or sublingually in the form of tablets, capsules, ovules, elixirs, solutions or suspensions, which may contain flavouring or colouring agents, for immediate-, delayed- or controlled-release applications. The agents of disclosure may also be administered via intracavernosal injection.

Such tablets may contain excipients such as microcrystalline cellulose, lactose, sodium citrate, calcium carbonate, dibasic calcium phosphate and glycine, disintegrants such as starch (preferably corn, potato or tapioca starch), sodium starch glycollate, croscarmellose sodium and certain complex silicates, and granulation binders such as polyvinylpyrrolidone, hydroxypropylmethylcellulose (HPMC), hydroxy-propylcellulose (HPC), sucrose, gelatin and acacia. Additionally, lubricating agents such as magnesium stearate, stearic acid, glyceryl behenate and talc may be included.

Solid compositions of a similar type may also be employed as fillers in gelatin capsules. Preferred excipients in this regard include lactose, starch, cellulose, milk sugar or high molecular weight polyethylene glycols. For aqueous suspensions and/or elixirs, the compounds of the invention may be combined with various sweetening or flavouring agents, colouring matter or dyes, with emulsifying and/or suspending agents and with diluents such as water, ethanol, propylene glycol and glycerin, and combinations thereof.

The agents of the invention can also be administered parenterally, for example, intravenously, intra-articularly, intra-arterially, intraperitoneally, intra-thecally, intraventricularly, intrasternally, intracranially, intra-muscularly or subcutaneously, or they may be administered by infusion techniques. They are best used in the form of a sterile aqueous solution which may contain other substances, for example, enough salts or glucose to make the solution isotonic with blood. The aqueous solutions should be suitably buffered (preferably to a pH of from 3 to 9), if necessary. The preparation of suitable parenteral formulations under sterile conditions is readily accomplished by standard pharmaceutical techniques well known to those skilled in the art.

Formulations suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilised) condition requiring only the addition of the sterile liquid carrier, for example water for injections, immediately prior to use. Extemporaneous injection solutions and suspensions may be prepared from sterile powders, granules and tablets of the kind previously described.

For oral and parenteral administration to human patients, the daily dosage level of the agents of the invention will usually be from 1 to 1000 mg per adult (*i.e*. from about 0.015 to 15 mg/kg), administered in single or divided doses.

The agents of the disclosure can also be administered intranasally or by inhalation and are conveniently delivered in the form of a dry powder inhaler or an aerosol spray presentation from a pressurised container, pump, spray or nebuliser with the use of a suitable propellant, *e.g.* dichlorodifluoromethane, trichlorofluoro-methane, dichlorotetrafluoro-ethane, a hydrofluoroalkane such as 1,1,1,2-tetrafluoroethane (HFA 134A3 or 1,1,1,2,3,3,3-heptafluoropropane (HFA 227EA3), carbon dioxide or other suitable gas. In the case of a pressurised aerosol, the dosage unit may be determined by providing a valve to deliver a metered amount. The pressurised container, pump, spray or nebuliser may contain a solution or suspension of the active compound, e.g. using a mixture of ethanol and the propellant as the solvent, which may additionally contain a lubricant, e.g. sorbitan trioleate. Capsules and cartridges (made, for example, from gelatin) for use in an inhaler or insufflator may be formulated to contain a powder mix of a compound of the invention and a suitable powder base such as lactose or starch.

Aerosol or dry powder formulations are preferably arranged so that each metered dose or 'puff' contains at least 1 mg of a compound of the invention for delivery to the patient. It will be appreciated that the overall daily dose with an aerosol will vary from patient to patient, and may be administered in a single dose or, more usually, in divided doses throughout the day.

Alternatively, the agents of the disclosure can be administered in the form of a suppository or pessary, or they may be applied topically in the form of a lotion, solution, cream, ointment or dusting powder. The compounds of the invention may also be transdermally administered, for example, by the use of a skin patch. They may also be administered by the ocular route.

For ophthalmic use, the agents of the disclosure can be formulated as micronised suspensions in isotonic, pH adjusted, sterile saline, or, preferably, as solutions in isotonic, pH adjusted, sterile saline, optionally in combination with a preservative such as a benzylalkonium chloride. Alternatively, they may be formulated in an ointment such as petrolatum.

For application topically to the skin, the agents of the disclosure can be formulated as a suitable ointment containing the active compound suspended or dissolved in, for example, a mixture with one or more of the following: mineral oil, liquid petrolatum, white petrolatum, propylene glycol, polyoxyethylene polyoxypropylene compound, emulsifying wax and water. Alternatively, they can be formulated as a suitable lotion or cream, suspended or dissolved in, for example, a mixture of one or more of the following: mineral oil, sorbitan monostearate, a polyethylene glycol, liquid paraffin, polysorbate 60, cetyl esters wax, cetearyl alcohol, 2-octyldodecanol, benzyl alcohol and water.

Formulations suitable for topical administration in the mouth include lozenges comprising the active ingredient in a flavoured basis, usually sucrose and acacia or tragacanth; pastilles comprising the active ingredient in an inert basis such as gelatin and glycerin, or sucrose and acacia; and mouth-washes comprising the active ingredient in a suitable liquid carrier.

Where the agent is a polypeptide, it may be preferable to use a sustained-release drug delivery system, such as a microspheres. These are designed specifically to reduce the frequency of injections. An example of such a system is Nutropin Depot which encapsulates recombinant human growth hormone (rhGH) in biodegradable microspheres that, once injected, release rhGH slowly over a sustained period.

Alternatively, polypeptide agents of the present disclosure can be administered by a surgically implanted device that releases the drug directly to the required site.

Electroporation therapy (EPT) systems can also be employed for the administration of proteins and polypeptides. A device which delivers a pulsed electric field to cells increases the permeability of the cell membranes to the drug, resulting in a significant enhancement of intracellular drug delivery.

Proteins and polypeptides can also be delivered by electroincorporation (EI). EI occurs when small particles of up to 30 microns in diameter on the surface of the skin experience electrical pulses identical or similar to those used in electroporation. In EI, these particles are driven through the stratum corneum and into deeper layers of the skin. The particles can be loaded or coated with drugs or genes or can simply act as "bullets" that generate pores in the skin through which the drugs can enter.

An alternative method of protein and polypeptide delivery is the thermo-sensitive ReGel injectable. Below body temperature, ReGel is an injectable liquid while at body temperature it immediately forms a gel reservoir that slowly erodes and dissolves into known, safe, biodegradable polymers. The active drug is delivered over time as the biopolymers dissolve.

Protein and polypeptide pharmaceuticals can also be delivered orally. One such system employs a natural process for oral uptake of vitamin B12 in the body to co-deliver proteins and polypeptides. By riding the vitamin B12 uptake system, the protein or polypeptide can move through the intestinal wall. Complexes are produced between vitamin B12 analogues and the drug that retain both significant affinity for intrinsic factor (IF) in the vitamin B12 portion of the complex and significant bioactivity of the drug portion of the complex.

Methods for administering oligonucleotide or polynucleotide are also well know in the art (see Dass, 2002, J Pharm Pharmacol. 54(1):3-27; Dass, 2001, Drug Deliv. 8(4):191-213; Lebedeva et al., 2000, Eur J Pharm Biopharm. 50(1):101-19; Pierce et al., 2005, Mini Rev Med Chem. 5(1):41-55; Lysik & Wu-Pong, 2003, J Pharm Sci. 2003 2(8):1559-73; Dass, 2004, Biotechnol Appl Biochem. 40(Pt 2):113-22; Medina, 2004, Curr Pharm Des. 10(24):2981-9.

The composition of the disclosure may further comprise at least one other agent.

Such a further agent may be an anti-inflammatory agent which includes but is not limited to non-steroidal anti-inflammatory agent (NSAID), a disease modifying anti-rheumatic drug (DMARD), a statin (including HMG-CoA reductase inhibitors such as simvastatin), a biological agent (biologicals), a steroid, an immunosuppressive agent, a salicylate and/or a microbicidal agent. Non-steroidal anti-inflammatory agents include anti-metabolite agents (such as methotrexate) and anti-inflammatory gold agents (including gold sodium thiomalate, aurothiomalate or gold salts, such as auranofin). Biologicals include anti-TNF agents (including adalimumab, etanercept, infliximab, anti-IL-1 reagents, anti-IL-6 reagents, anti-B cell reagents (retoximab), anti-T cell reagents (anti-CD4 antibodies), anti-IL-15 reagents, anti-CLTA4 reagents, anti-RAGE reagents), antibodies, soluble receptors, receptor binding proteins, cytokine binding proteins, mutant proteins with altered or attenuated functions, RNAi, polynucleotide aptemers, antisense oligonucleotides or omega 3 fatty acids. Steroids (also know as corticosteroids) include cortisone, prednisolone or dexamethasone. Immunosuppressive agents include cylcosporin, FK506, rapamycin, mycophenolic acid. Salicylates include aspirin, sodium salicylate, choline salicylate and magnesium salicylate. Microbicidal agents include quinine and chloroquine. For example, the agent may be administered in combination with one or more of an NSAID, DMARD, or immunosuppressant

Also provided is an agent or composition according to the present disclosure for use as a medicament, in particular for use in the treatment of a chronic inflammatory condition.

A chronic inflammatory condition is any condition associated with inappropriate inflammation. Such conditions include, but are not limited to, rheumatoid arthritis (RA), autoimmune conditions, inflammatory bowel diseases, non-healing wounds, multiple sclerosis, cancer, atherosclerosis, sjogrens disease, diabetes, lupus erythrematosus (including systemic lupus erythrematosus), asthma, fibrotic diseases (including liver cirrhosis), pulmonary fibrosis, UV damage and psoriasis.

### Definitions

By "inflammation" we include the meaning of local accumulation of fluid, plasma proteins, and white blood cells that is initiated by tissue injury, infection or a local immune response.

By "acute inflammation" we include the meaning of the initial stages (initiation) of inflammation and the short-term transient inflammatory response immediately after injury, infection or local immune response. Typically, acute inflammation is rapidly resolved, lasting from a matter of minutes to no longer that a few days.

By "chronic inflammation" we include the meaning of persistent and/or non-resolved inflammation. It is often associated with inappropriate destruction of healthy tissue. This may be progressive and last over a period of weeks or longer. Chronic inflammation is typically associated with persistent infection or disease including, but not limited to, automimmune conditions.

By "chronic joint inflammation" we include the meaning of persistent inflammation that is progressive and unremitting over a period of weeks to months, resulting in distortion of the affected joint and radiographic evidence of cartilage and bone destruction as observed in human disease (Kelly, Harris, Ruddy and Sledge, Textbook of Rheumatology 4th Edition).

In experimental murine models, chronic joint inflammation is characterised by inflammation that does not subside and causes inappropriate tissue destruction, even over a relatively short period of time. This is characterized (and can be identified) histologically by the prolonged presence of inflammatory cells in the synovium and joint space, chondrocyte death, and cartilage and bone erosion.

By "fragment" we mean at least 10 nucleotides, for example at least 15, 16, 17, 18, 19, 20, 21, 22, 23, 24 or 25 nucleotides.

By "variant" we mean that the nucleotide sequence shares at least 90% sequence identity with the full length sequence of interest, for example at least 50%, 55%, 60%, 65%, 70%, 75%, 80%, 85%, 90%, 95%, 96%, 97%, 98% or 99% sequence identity.

The percent sequence identity between two polynucleotides may be determined using suitable computer programs, for example the GAP program of the University of Wisconsin Genetic Computing Group and it will be appreciated that percent identity is calculated in relation to polynucleotides whose sequences have been aligned optimally.

The alignment may alternatively be carried out using the Clustal W program (as described in Thompson et al., 1994, Nuc. Acid Res. 22:4673-4680).

The parameters used may be as follows:
Fast pairwise alignment parameters: K-tuple(word) size; 1, window size; 5, gap penalty; 3, number of top diagonals; 5. Scoring method: x percent.
Multiple alignment parameters: gap open penalty; 10, gap extension penalty; 0.05.
Scoring matrix: BLOSUM.

Alternatively, the BESTFIT program may be used to determine local sequence alignments.

By "antibody" we include substantially intact antibody molecules, as well as chimaeric antibodies, humanised antibodies, human antibodies (wherein at least one amino acid is mutated relative to the naturally occurring human antibodies), single chain antibodies, bispecific antibodies, antibody heavy chains, antibody light chains, homodimers and heterodimers of antibody heavy and/or light chains, and antigen binding fragments and derivatives of the same.

By "antigen-binding fragment" we mean a functional fragment of an antibody that is capable of binding to tenascin-C.

The term "subject" means all animals including humans. Examples of subjects include humans, cows, dogs, cats, goats, sheep, and pigs. The term "patient" means a subject having a disorder in need of treatment.

As used herein, 'pharmaceutical formulation' means a therapeutically effective formulation according to the invention.

A 'therapeutically effective amount', or 'effective amount', or 'therapeutically effective', as used herein, refers to that amount which provides a therapeutic effect for a given condition and administration regimen. This is a predetermined quantity of active material calculated to produce a desired therapeutic effect in association with the required additive and diluent, *i.e.* a carrier or administration vehicle. Further, it is intended to mean an amount sufficient to reduce and most preferably prevent, a clinically significant deficit in the activity, function and response of the host. Alternatively, a therapeutically effective amount is sufficient to cause an improvement in a clinically significant condition in a host. As is appreciated by those skilled in the art, the amount of a compound may vary depending on its specific activity. Suitable dosage amounts may contain a predetermined quantity of active composition calculated to produce the desired therapeutic effect in association with the required diluent. In the methods and use for manufacture of compositions of the invention, a therapeutically effective amount of the active component is provided. A therapeutically effective amount can be determined by the ordinary skilled medical or veterinary worker based on patient characteristics, such as age, weight, sex, condition, complications, other diseases, *etc.,* as is well known in the art.

Examples embodying an aspect of the disclosure will now be described with reference to the following figures in which:

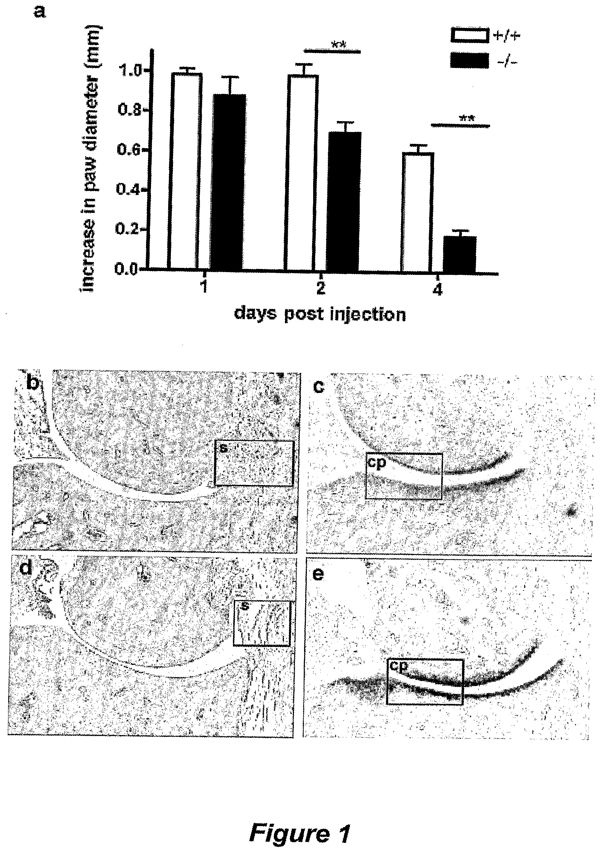

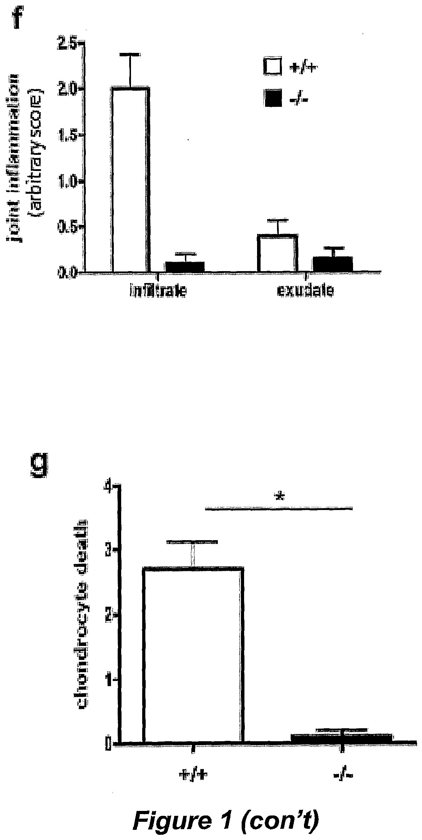

**Figure 1****. Accelerated resolution of acute inflammation in tenascin-C deficient mice.** (a) Paw swelling in wild type (+/+)(white bars) and tenascin-C null (-/-)(black bars) mice over time after injection of zymosan. Data are shown as the mean increase in paw diameter compared to paw diameter before injection +/-SEM (n = 24 mice per genotype). **= p<0.01. (b-e) Representative sections of the ankle joint from wild type (b, c) and tenascin-C null (d, e) mice 4 days after zymosan injection, stained with hemotoxylin and eosin (b, d) and safranin-O (c, e). Boxes highlight the joint synovium (s) and cartilage proteoglycan (cp). Magnification x10. Quantification of joint inflammation (f) and chondrocyte death (g) in knee joints 4 days after injection with zymosan from wild type mice (white bars) and tenascin-C null mice (black bars). Data are expressed as the mean (+/- SD) (n = 24 mice per genotype). *= p <0.05.

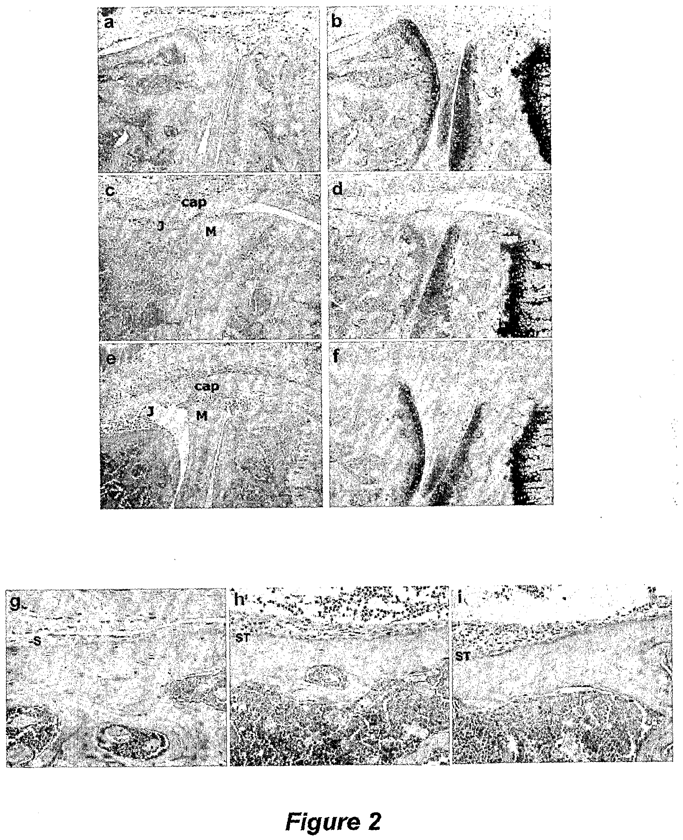

**Figure 2****. Synovial inflammation is induced in tenascin-C deficient mice upon injection of antigen.** (a-b, g) Representative sections of the knee joint of sham injected wild type mice. (c-f, h-i) Representative sections of the knee joint of wild type (c, d, h) or tenascin-C null (e, f, i) mice 24 hours after intra-articular injection of mBSA. Inflammatory cell infiltration in the capsule, meniscus and the joint space of both wild type and tenascin-C null mice is highlighted by (cap), (M) and (J) respectively. (S) highlights the healthy synovium of sham injected mice that is no more than 1-3 cells thick along the entire bone surface and (ST) highlights the synovia of wild type and tenascin-C null mice which are both significantly thickened. Sections are stained with hemotoxylin and eosin (a, c, e, g, h, i) and safranin-O (b, d, f). Magnification x10 (a-f) or x40 (g-i). (n = 5 mice per genotype).

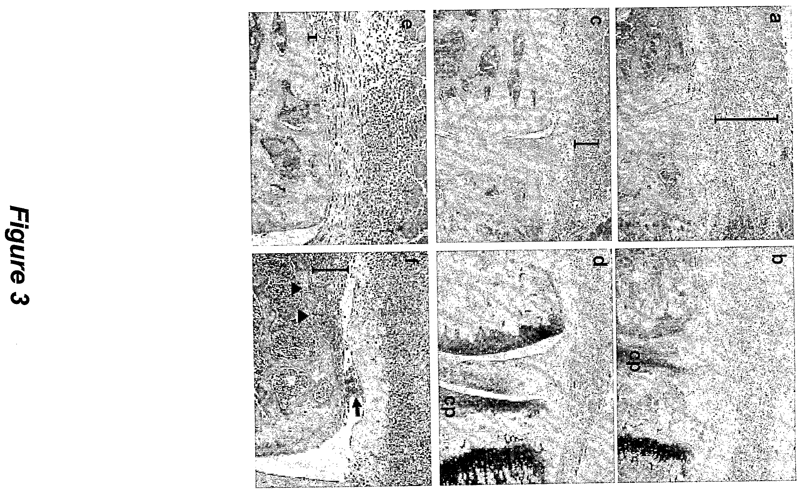

**Figure 3****. Synovial inflammation subsides rapidly in tenascin-C deficient mice.** Representative sections of the knee joint of wild type (a, b, f) or tenascin-C null (c, d, e) mice 3 days after intra-articular injection of mBSA. (a, c) The line highlights increased inflammation of the capsule in wild type mice compared to tenascin-C null mice. (b, d) (cp) highlights increased cartilage proteoglycan loss in wild type mice compared to tenascin-C null mice. (e, f) Significant synovial hyperplasia (line), cell and fibrin deposits in the joint space (arrow) and pannus invasion (arrow heads) are observed in wild type mice compared to tenascin-C null mice. Sections are stained with hemotoxylin and eosin (a, c, e, f) and safranin-O (b, d) Magnification x10 (a-d) or x20 (e-f). (n = 5 mice per genotype).

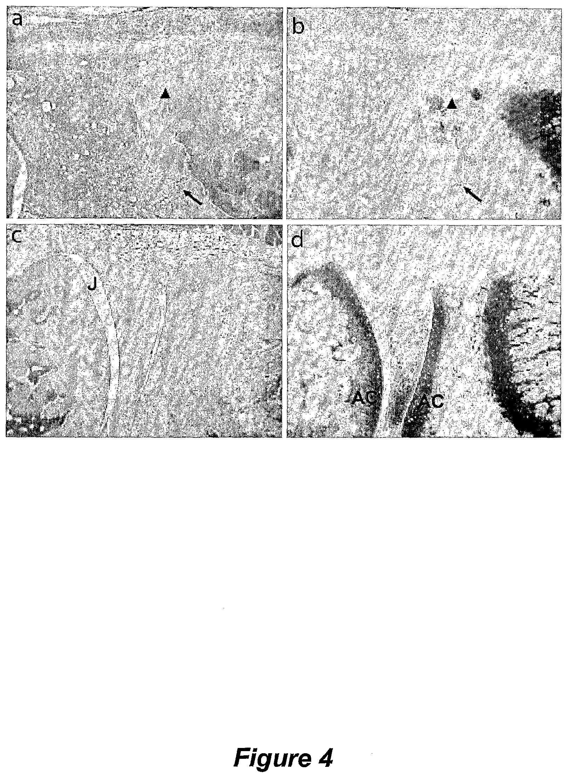

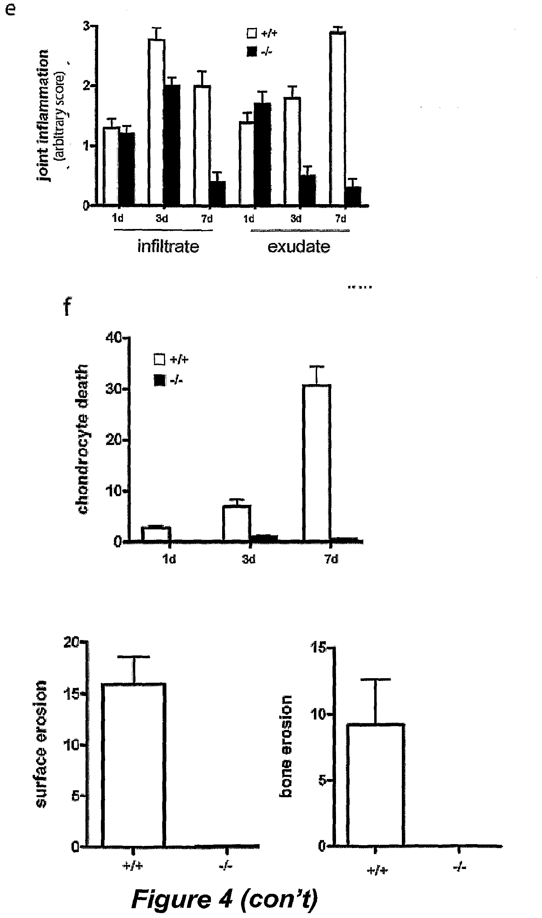

**Figure 4****. tenascin-C deficient mice are protected from tissue destruction during antigen induced arthritis.** (a-b) Representative sections of the knee joint of wild type mice 7 days after intra-articular injection of mBSA, stained with hemotoxylin and eosin (a) and safranin-O (b). Magnification x10. (n = 24 mice per genotype). Arrowhead highlights area of bone erosion. Arrow highlights pannus invasion into articular cartilage. (c-d) Representative sections of the knee joint of tenascin-C null type mice 7 days after intra-articular injection of mBSA, stained with hemotoxylin and eosin (c) and safranin-O (d). Magnification x10. (n = 24 mice per genotype). J highlights the joint space and AC the intact articular cartilage. (e) Histological score of knee joint inflammation 24 hours, 3 days and 7 days after injection with mBSA from wild type mice (white bars) and tenascin-C null mice (black bars). Data represent the mean +/- SD (n = 5 per genotype (24h, 3d) or 24 per genotype (7d)). (f) Quantification of chondrocyte death, cartilage surface erosion and bone erosion after injection with mBSA in knee joints from wild type mice (white bars) and tenascin-C null mice (black bars). Chondrocyte death is shown at 24 hours, 3 days and 7 days, and cartilage surface erosion and bone erosion at 7d. Data represent the mean +/- SD (n = 5 per genotype (24h, 3d) or 24 per genotype (7d)).

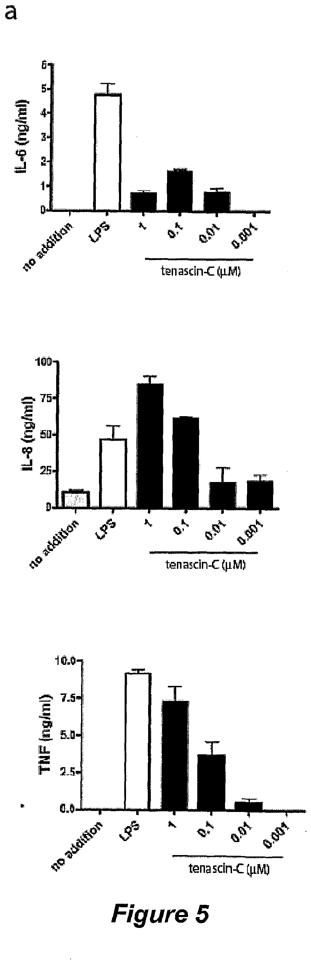

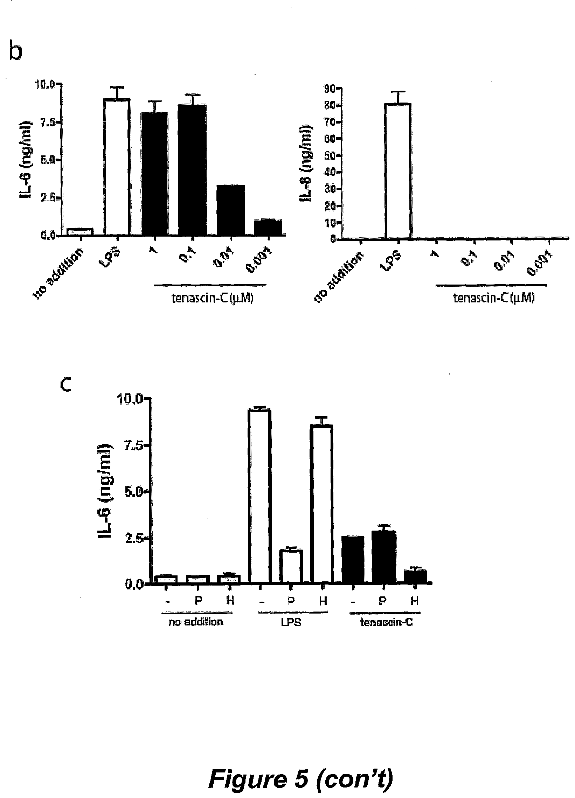

**Figure 5****. tenascin-C induces TNF-α, IL-6 and IL-8 synthesis in primary human macrophages and RA synovial fibroblasts.** (a-b) Primary human macrophages (a) and RA synovial fibroblasts (b) were unstimulated (no addition) or stimulated with LPS (1 ng/ml (a) or 10 ng/ml (b)) or recombinant tenascin-C (1.0 µM - 1.0 nM) for 24h. Data shown are the mean of triplicate values (+/-SD) from one of three representative experiments. (c) Primary human macrophages were unstimulated (no addition) or stimulated with LPS (1 ng/ml) or recombinant tenascin-C (1.0 µM) for 24h. (-) indicates cells were pre-incubated with medium alone. (P) Cells were pre-incubated with 25 µg/ml polymyxin B for 30 min before stimulation. (H) Cells were incubated with medium with no addition or containing LPS or tenascin-C that was boiled for 15 minutes before addition to cells. Data shown are the mean of triplicate values (+/- SD) from one of three representative experiments.

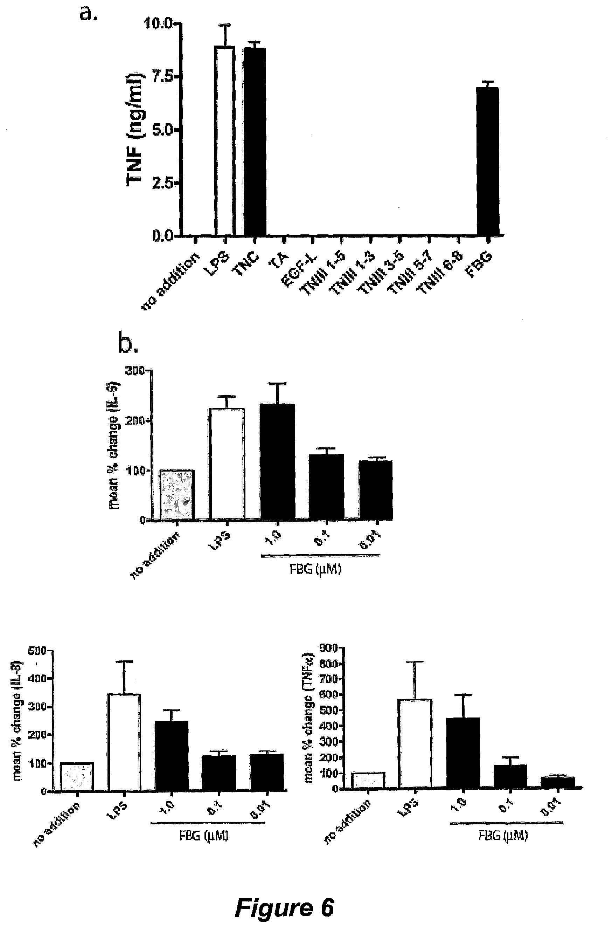

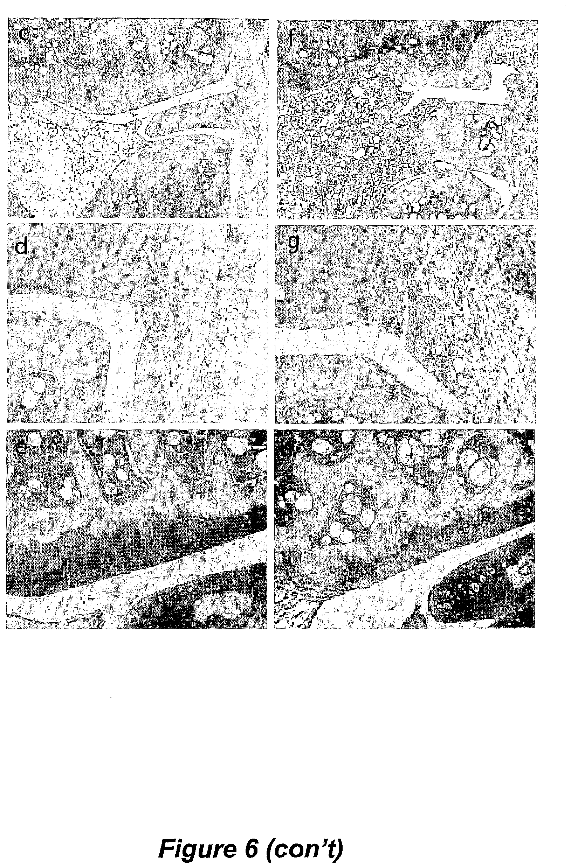

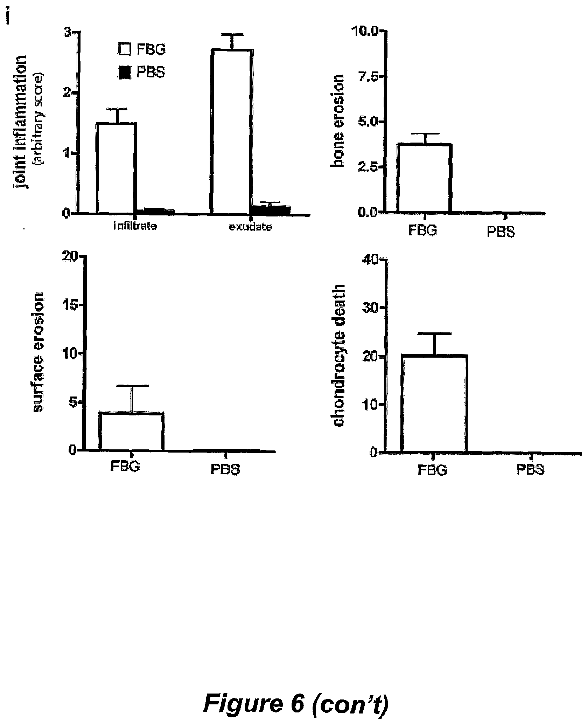

**Figure 6****. The FBG domain of tenascin-C mediates stimulation of cytokine synthesis *in vivo* and *in vitro.*** (a) Primary human macrophages were unstimulated (no addition) or stimulated with LPS (1 ng/ml), recombinant tenascin-C (TNC) or 1.0 µM tenascin-C domains (TA, EGF-L, TNIII1-5, TNIII1-3, TNIII3-5, TNIII5-7, TNIII6-8 and FBG) for 24h. Data shown are the mean of triplicate values (+/- SD) from one of three representative experiments. (b) RA synovial membrane cells were unstimulated (no addition) or stimulated with LPS (10 ng/ml) or recombinant FBG (1.0 - 0.01 µM) for 24h. Data shown are the mean % change in cytokine levels compared to unstimulated cells (+/- SEM) from five different patients. (c-h) Representative sections of the knee joint of wild type mice 3 days after intra-articular injection of PBS (c-e) or 1 µg FBG (f-h). Sections are stained with hemotoxylin and eosin (c,d,f,g) or Safranin-O (e, h). Magnification x10 (c, f) or x25 (d,e,g,h) (n = 5 mice per genotype). (i) Quantification of joint inflammation, bone erosion, cartilage surface erosion and chondrocyte death in the knee joints of wild type mice 3 days after intra-articular injection of PBS (black bars) or 1 µg FBG (white bars). Data represent the mean +/- SD (n = 5 per genotype).

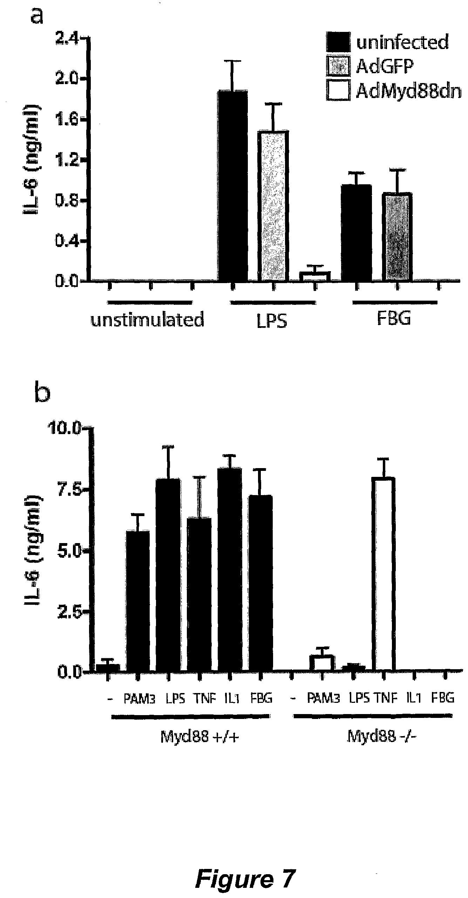

**Figure 7****. FBG mediated cytokine synthesis is MyD88 dependent.** (a) Human RA synovial fibroblasts were either uninfected, infected with adenovirus expressing GFP alone (AdGFP) or infected with adenovirus expressing dominant negative MyD88 (AdMyD88dn). Cells were unstimulated, stimulated with LPS (10 ng/ml) or stimulated with FBG (1µM) for 24h. Data shown are the mean of three independent experiments (+/- SEM). (b) Mouse embryonic fibroblasts isolated from wild type (+/+) or MyD88 deficient (-/-) mice were unstimulated (-) or stimulated with PAM3 (100 ng/ml), LPS (100 ng/ml), TNFα (100 ng/ml), IL-1 (5 ng/ml) and FBG (1 µM) for 24h. Data shown are the mean of three independent experiments (+/-SEM).

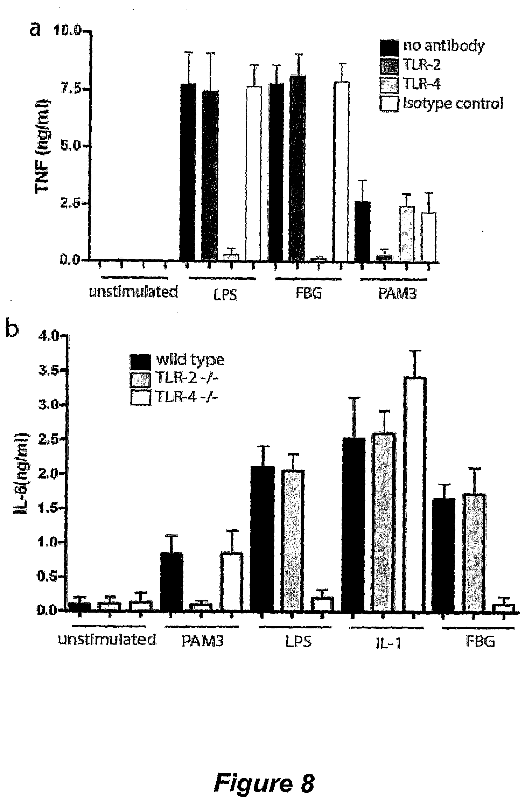

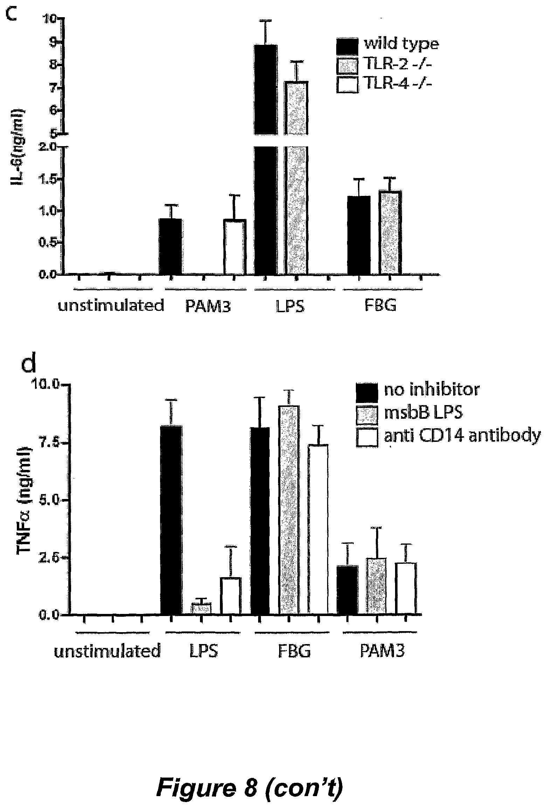

**Figure 8****. FBG mediated cytokine synthesis is TLR4 dependent but does not require CD14 or MD-2.** (a) Primary human macrophages were pre-incubated with medium alone or medium containing function blocking antibodies to TLR2 (10 µg/ml), TLR4 (25 µg/ml) or isotype control antibodies (25 µg/ml) for 30 min before stimulation. Cells were unstimulated, or stimulated with LPS (1 ng/ml), FBG (1 µM) or PAM3 (10 ng/ml) for 24h. Data shown are the mean of three independent experiments (+/-SEM). (b) Mouse embryonic fibroblasts isolated from wild type, TLR2 (TLR2 -/-) or TLR4 (TLR4 -/-) deficient mice were unstimulated or stimulated with PAM3 (100 ng/ml), LPS (100 ng/ml), IL-1 (5 ng/ml) and FBG (1 µM) for 24h. Data shown are the mean of three independent experiments (+/-SEM). (c) Bone marrow derived macrophages isolated from wild type, TLR2 (TLR2 -/-) or TLR4 (TLR4 -/-) deficient mice were unstimulated or stimulated with PAM3 (100 ng/ml), LPS (100 ng/ml) or FBG (1 µM) for 24h. Data shown are the mean of three independent experiments (+/-SEM). (d) Human macrophages were pre-incubated with no inhibitor, 1µg/ml msbB LPS or 10µg/ml anti-CD14 antibody for 30 min before stimulation with LPS (1 ng/ml), FBG (1 µM) or PAM3 (10 ng/ml) for 24h. Data shown are the mean of three independent experiments (+/-SEM).

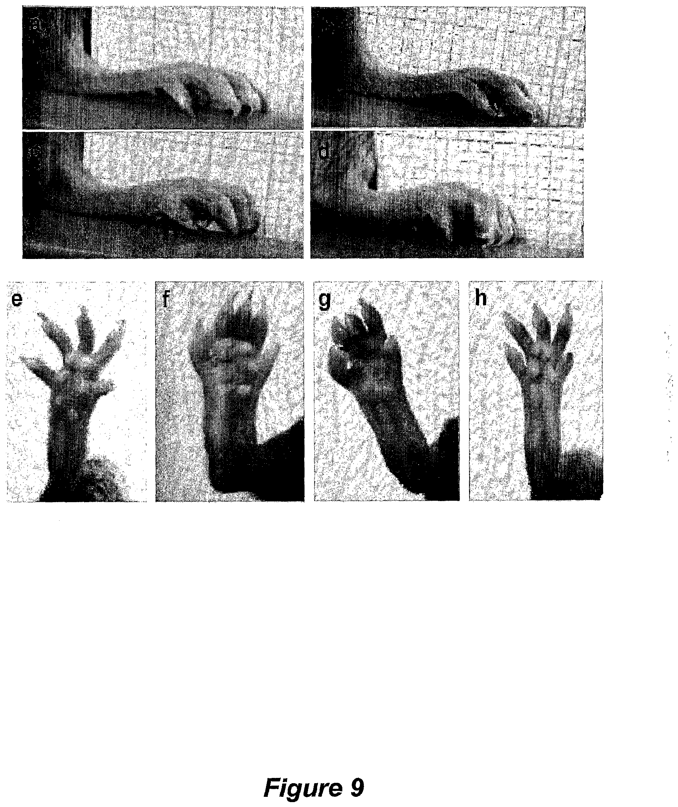

**Figure 9****. Paw swelling over time after injection of zymosan.** Representative images of the paws of non-injected tenascin-C null mice (a, e) (diameter 1.6 mm), tenascin-C null mice 24h (d, f) (diameter 2.5 mm) and 4d (b, h) (diameter 1.7 mm) after zymosan injection and from wild type mice 4d after zymosan injection (c, g) (diameter 2.1 mm).

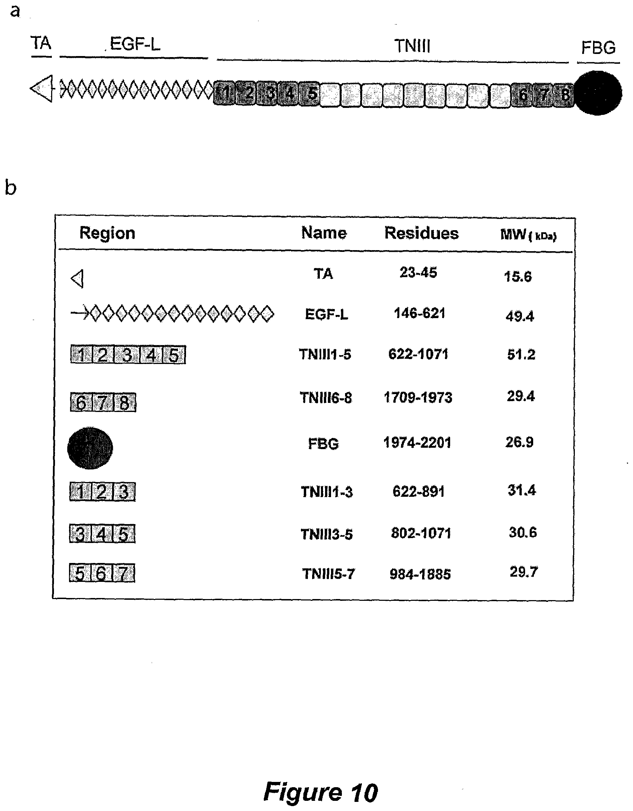

**Figure 10****. Synthesis of recombinant proteins.** (a) Domain structure of the tenascin-C monomer comprising different domains, including the assembly domain (TA), 14 and a half EGF-like repeats (EGF-L), 17 fibronectin type III-like repeats (TNIII) (8 constitutively expressed (1-8) and 9 that can be alternatively spliced, and a fibrinogen-like globe (FBG). (b) The regions covered by the recombinant proteins that were synthesized, the corresponding amino acid residues and the molecular weight of each protein.

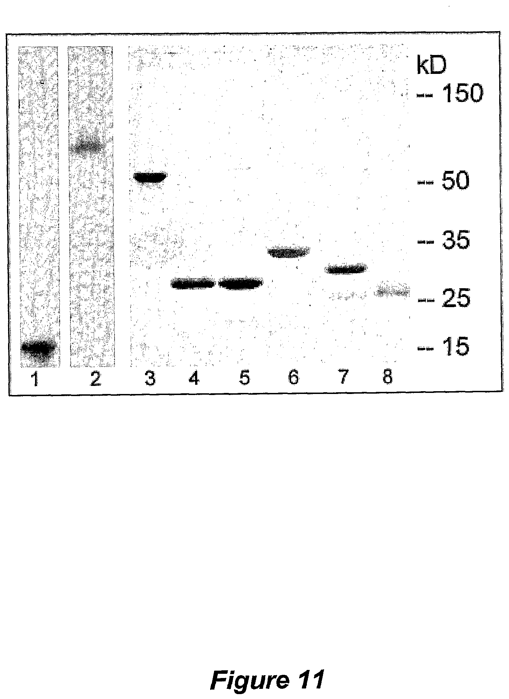

**Figure 11****. Analysis of protein purity.** Silver stained gel showing 1 µg of each recombinant protein analysed by SDS-PAGE under reducing conditions. Lanes: 1 (TA), 2 (EGF-L), 3 (TNIII1-5), 4 (TNIII5-7), 5 (TNIII6-8), 6 (TNIII1-3), 7 (TNIII3-5) and 8 (FBG).

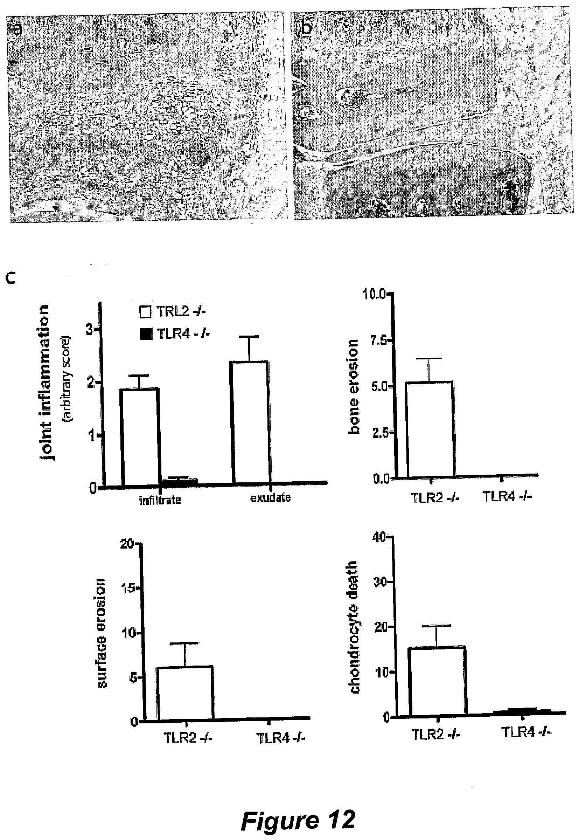

**Figure 12****. FBG-mediated joint inflammation in vivo requires expression of TLR4.** Representative sections of the knee joint of TLR2 (a) and TLR4 (b) null mice 3 days after intra-articular injection of 1 µg FBG. Sections are stained with hemotoxylin and eosin. Magnification x10 (n = 5 mice per genotype). (c) Quantification of joint inflammation, bone erosion, cartilage surface erosion and chondrocyte death in the knee joints of TLR2 (white bars) and TLR4 (black bars) null mice 3 days after intra-articular injection of 1 µg FBG. Data represent the mean +/- SD (n = 5 per genotype).
**Figure 13****. Amino acid sequence of human tenascin-C and its domains**

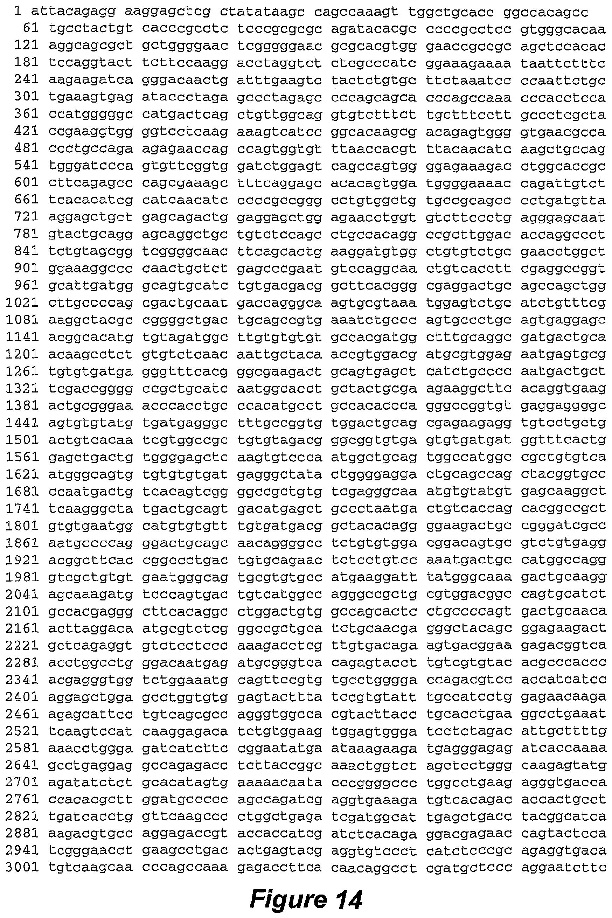

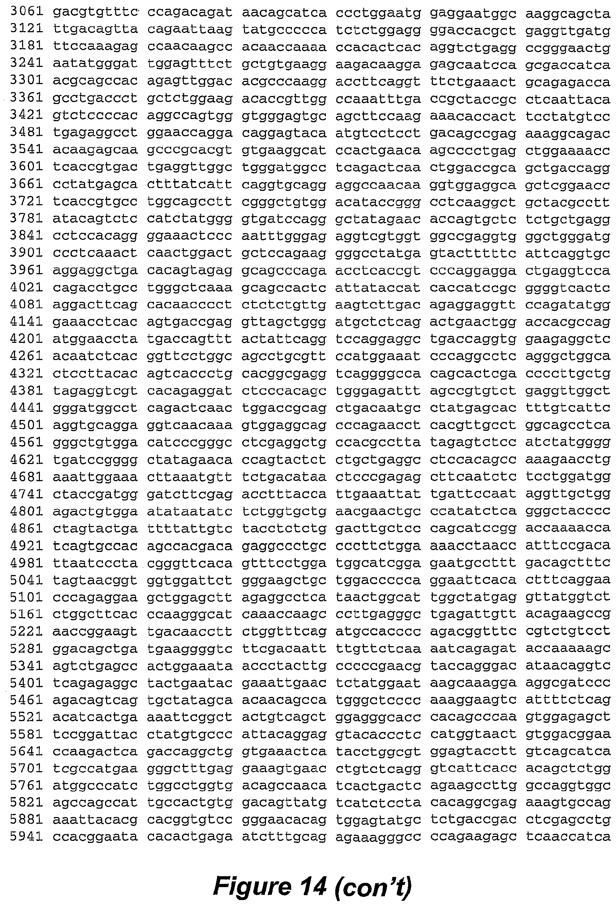

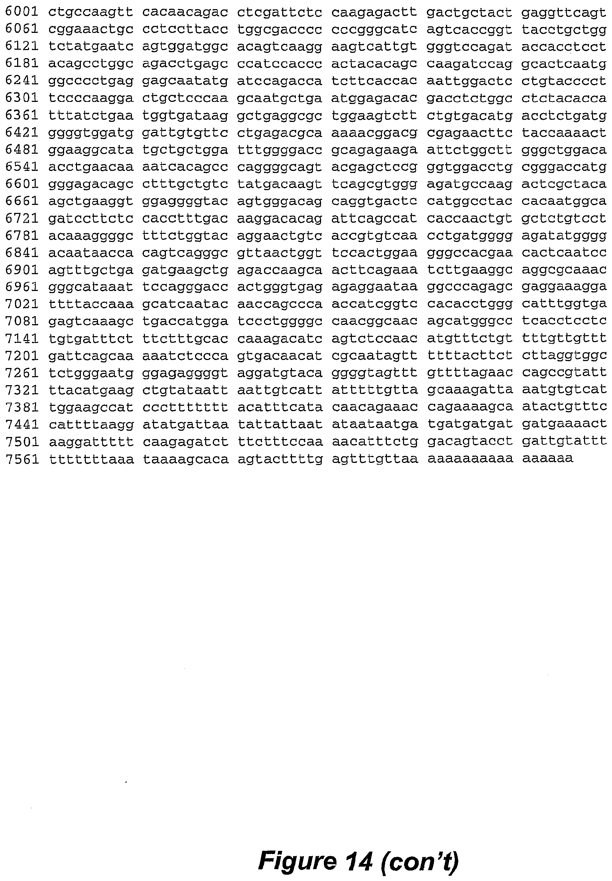

**Figure 14****. Nucleotide sequence of human tenascin-C**

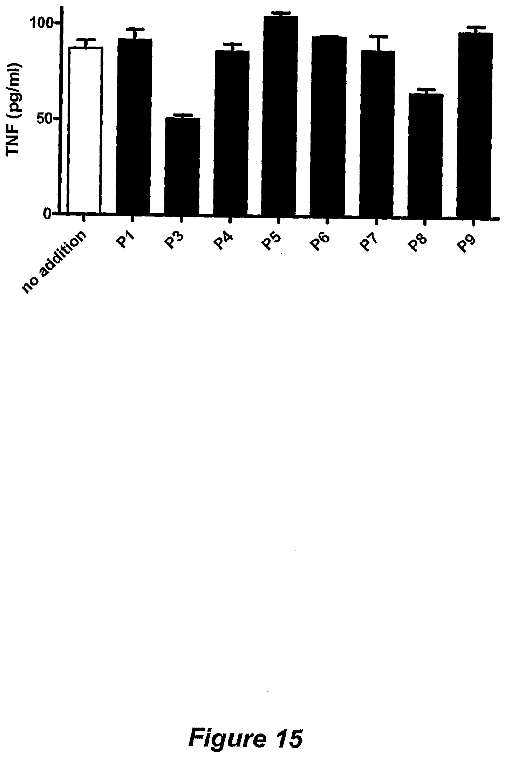

**Figure 15****. TNF synthesis in response to specific FBG peptides.** TNF synthesis by RA membrane cultures incubated for 24h with no addition or 100 µM of each FBG peptide (P1, P3-P9).

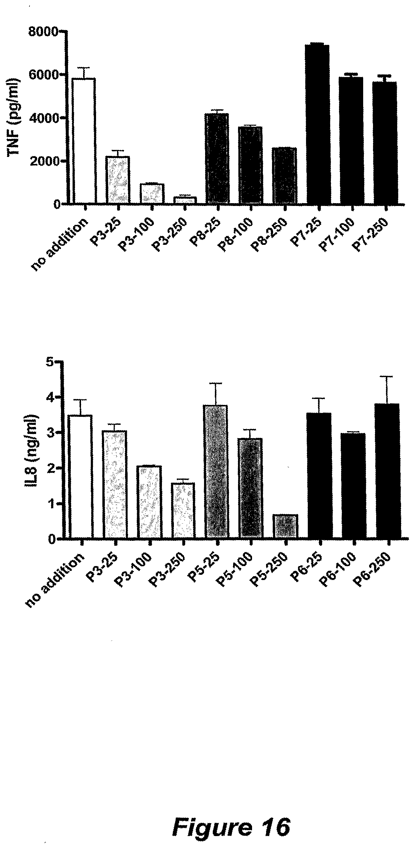

**Figure 16****. TNF and IL8 synthesis in response to varying concentrations of specific FBG peptides.** TNF & IL8 synthesis by RA membrane cultures incubated for 24h with no addition or 25, 100 or 250 µM of FBG peptide.

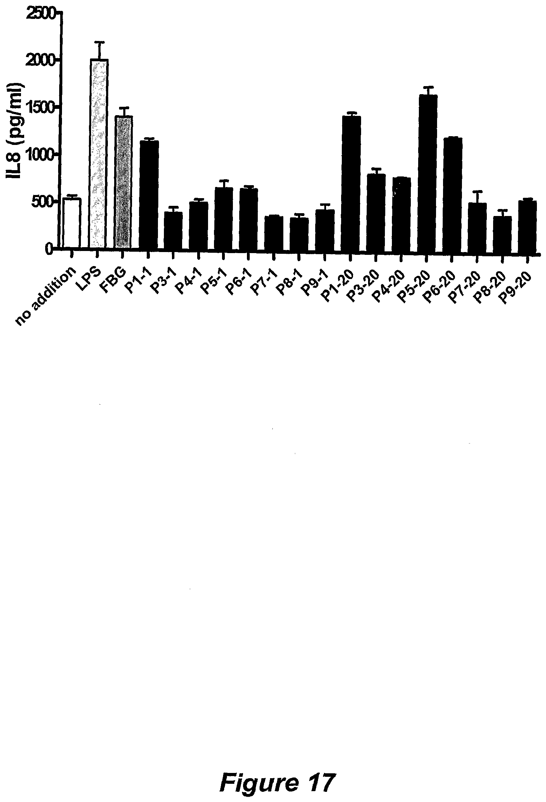

**Figure 17****. IL8 synthesis in response to LPS, whole FBG domain or specific FBG peptides.** IL8 synthesis by macrophages after 24h incubation with no addition, 1 ng/ml LPS, 1 µM whole FBG domain (FBG) or 1 or 20 µM of FBG peptides (P1, P3-P9).

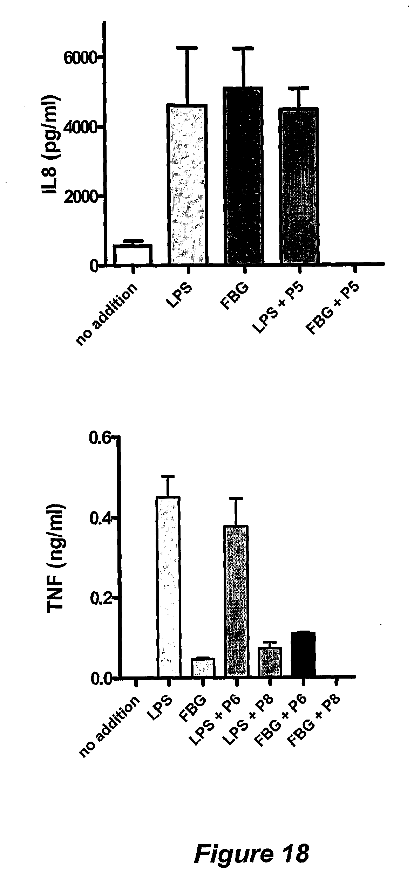

**Figure 18****. IL8 and TNF synthesis in response to LPS and FBG following pre-incubation with FBG peptides.** TNF and IL8 synthesis by macrophages after 24h incubation with no addition, 1 ng/ml LPS or 1 µM whole FBG domain (FBG), either with or without pre-incubation with 20 µM of FBG peptides.

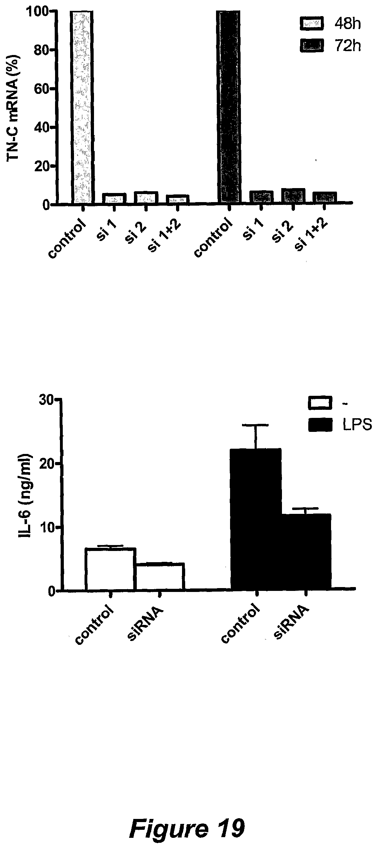

**Figure 19****. IL8 and TNF synthesis in response to tenascin-C targeted siRNAs.** Tenascin-C mRNA levels in RA fibroblasts transfected with luciferase specific siRNA (control), or with tenascin-C targeted siRNAs: oligo 1 (si 1), oligo 2 (si 2) or a combination of oligos 1+2 (si 1+2). IL6 synthesis in RA fibroblasts transfected with luciferase siRNA (control) or with a combination of tenascin-C targeted oligos 1+2 (siRNA) in the presence or absence of 10 ng/ml LPS for 24h.

### Example 1 -General methods

### Reagents

Zymosan, methylated BSA and Freund's complete adjuvant, anti-FLAG M2 antibody (mouse monoclonal antibody), blasticidin, and isotype control antibodies (Mouse IgG2a, IgG1) were from Sigma-Aldrich (Dorset, UK). Hypnorm was from VetaPharma Ltd. (Leeds, UK). The *Limulus amaebocyte* lysate assay was from Associates of Cape Cod (Liverpool, UK). Wild type human embryonic kidney (HEK293-EBNA) cells were from Invitrogen (Groningen, Netherlands). M-CSF and murine IL-1β were from PeproTech (Neuilly-Sur-Seine, France). DMEM, RPMI 1640, fetal bovine serum (FBS), penicillin/streptomycin, antibiotic-antimycotic solution PSA and β-Mercaptoethanol were from PAA Laboratories (Yeovil, UK). HEK293 cell lines stably expressing human TLR2 and TLR4/CD14/MD-2, polymyxin B, msbB LPS and the function blocking TLR2 (*Clone:* TL2.1 *Isotype:* Mouse IgG2a) and TLR4 antibodies (*Clone:* HTA125 *Isotype:* Mouse IgG2a) were from Invivogen (Calne, UK). Phenol-chloroform-purified *Escherichia coli* LPS (rough and smooth) and Pam3Cys-Ser-Lys4 (Pam3C) were from Alexis (Birmingham, UK). Murine TNF-α and IL-1 receptor antagonist (IL-1ra-IL-1F3) were from R&D Systems (Abingdon, UK). Function blocking anti-CD14 antibodies (*Isotype:* Mouse IgG1) were from Abcam (Cambridge, UK). Human and murine TNF-α, IL-6, and IL-8 ELISAs were from Pharmingen (Oxford, UK).

### Purification of full-length tenascin-C

To ensure that cytokine production was not attributed to bacterial contaminants such as LPS and LPS-associated molecules we purified recombinant full-length human tenascin-C from the conditioned medium of the mammalian cell line HEK293 transfected with his-tagged human tenascin-C in the pCEP-pu vector as described (Lange (2007)). tenascin-C was purified to homogeneity as described (Lange (2007) and determined to be free of LPS contamination using the *Limulus amaebocyte* lysate assay according to the manufacturer's instructions.

### Synthesis of recombinant proteins

Proteins corresponding to each domain of tenascin-C were synthesized (TA, EGF-L, various TNIII repeats and FBG) and purified. See Example 2.

### Measurement of LPS Contamination in Recombinant Proteins

To ascertain the levels of LPS in each recombinant protein the *Limulus amaebocyte* lysate assay was used according to the manufacturer's instructions (sensitivity ∼0.7 ± 0.5 pg LPS per mg protein). All recombinant proteins used in this study had levels of LPS that were less than 10pg/ml.

### Adenoviral Vectors and Their Propagation

Recombinant, replication-deficient adenoviral vectors encoding wild type MyD88 (AdMyD88wt), dominant-negative forms of MyD88 (AdMyD88dn) and the GFP control (AdGFP) were constructed in-house. A description of the synthesis of these viruses is in Andreakos (2004). All viruses used in this study are E1/E3 deleted, belong to the Ad5 serotype. Viruses were propagated in 293 human embryonic kidney cells, purified by ultracentrifugation through two cesium chloride gradients, and viral titers determined by plaque assay as previously described (Sacre (2007)).

### Animals

Homozygous tenascin-C deficient mice from the original stock described by Saga (1992) on a 129/sv an inbred strain of mice with a white bellied and agouti appearance background were provided by Prof. Charles French-Constant (University of Edinburgh, UK). Age matched congenic inbred wild type 129/sv mice were obtained from Charles River (Margate, UK). All tenascin-C deficient and wild type 129/sv mice were male and between 8-10 weeks of age at the time of experimentation.

Homozygous TLR2 and TLR4 deficient mice on a C57BL/6 background (an inbred strain of mice with a black coat) were obtained from B&K Universal (Hull, UK) Hoshino (1999) and Takeuchi (1999). Homozygous MyD88 deficient mice on a C57BL/6 background were provided by the Sanger Institute (Cambridge, UK). Age matched congenic inbred wild type C57B/L6 mice were obtained from Charles River (Margate, UK). For isolation of mouse embryo fibroblasts one female aged 8-10 weeks was mated with two males aged 8-10 weeks. For isolation of bone marrow derived macrophages mice were female and between 10-12 weeks of age at the time of experimentation.

All animals were fed standard rodent chow and water ad libitum, and were housed (<6 mice/cage) in sawdust-lined cages in an air-conditioned environment with 12-hour light/dark cycles. All animal procedures were approved by the institutional ethics committee.

### Statistical Methods

Mean, SD, SEM, and statistical tests were calculated using GraphPad version 3 (GraphPad Software Inc., San Diego, CA). Multiple group means were analyzed by one-way analysis of variance, followed by the Dunnett Multiple Comparisons test, where appropriate. Unpaired t-test was used for experiments involving only two groups.

### Example 2 - Synthesis of recombinant proteins

Proteins corresponding to each domain of tenascin-C were synthesized (TA, EGF-L, various TNIII repeats and FBG) and purified. The recombinant proteins synthesized are depicted in Figure 9.

### Reagents

Pfu Turbo polymerase was from Stratagene (Amsterdam, Netherlands). Easy mix 50 PCR tubes were from Molecular Bioproducts (Lutterworth, UK). RNeasy kits and Ni²⁺-NTA-agarose columns were from Qiagen (Crawley, UK). pCR Blunt vector, pCEP4 plasmid vector, human embryonic kidney (HEK293-EBNA) cells and 4-12% Bis-Tris gradient gels were from Invitrogen (Groningen, Netherlands). pET32b vector and BL21 (DE3) Rosetta cells were from Novagen (Kent, UK). HiTrap Q columns, HiTrap S columns, Sephacryl S500 HR column and heparin sepharose columns were from Amersham (Buckinghamshire, UK).

Restriction enzymes were obtained from New England BioLabs (Hitchin, UK). DMEM, fetal bovine serum (FBS) and penicillin/streptomycin were from PAA laboratories (Yeovil, UK). FuGENE6 transfection reagent was from Roche Applied Science (Basel, Switzerland).

Anti-FLAG M2 antibody (mouse monoclonal antibody), anti-FLAG M2-agarose, FLAG peptide were from Sigma-Aldrich (Dorset, UK). Anti-tetra-his antibody (mouse monoclonal antibody) was from Qiagen (Crawley, UK). Alkaline phosphatase-conjugated goat anti-(mouse IgG) IgG and Western Blue stabilized substrate for alkaline phosphatase were from Promega (Southampton, UK). Precision Protein Standards for SDS-PAGE were from BioRad (Hemel Hempstead, UK).

### Primer design

Domain boundaries were determined using alignments published in the human tenascin-C sequence (Siri (1991) accession number P24821 (Swiss-Prot)). To clone each domain we designed PCR primers where both the forward and reverse primers contained 18-21 bases corresponding to the 5' and 3' terminal sequences of the requisite coding sequence. The forward primer contained an Nde1 restriction site, followed by an N terminal his tag, immediately before the coding sequence. The final 3 bases of the Nde1 site form the ATC methionine initiation code. The reverse primer included a TTA stop codon immediately after the coding sequence, followed by a BamH1 or a Kpn1 site to allow unidirectional cloning into pET32b expression vectors.

**Table 1**

| Protein name | Forward primer |
|---|---|
| | Reverse primer |
| TA | FW: ATA***CATATG***CATCATCATCATCATCATGGGGTCCTCAAG AAAGTCATCCGG |
| | RV: GCC***GGATCC***TTAGCCTGCTCCTGCAGTACATTG |
| EGF-L | PCR1 |
| | FW: ACAGT***GGTACC***ACCATGGGGGCCATGGGGGCCATGACT CAGCTGTTG |
| | RV: **CTTGTCATCGTCGTCCTTGTAGTCACC**TTCGGTAGCGAG GGCAAG |
| | PCR2 |
| | FW: **GACTAGAAGGACGACGATGACAAG**TGCTGTCTCCAGCC TGCCAC |
| | RV: GACAGC***GGATCC***TTAATGATGATGATGATGATGTGAGCA GTCTTCTCCGCTGTAGC |
| TN1-5 | FW: ATA***CATATG***CATCATCATCATCATCATGAGGTGTCTCCTCC CAAAGA |
| | RV: GCC***GGTACC***TTAAGTGGATGCCTTCACACGTGC |
| TN1-3 | FW: ATA***CATATG***CATCATCATCATCATCATGAGGTGTCTCCTC CCAAAGA |
| | RV: GCC***GGTACC***TTATGTTGTGAAGGTCTCTTT GGC |
| TN3-5 | FW: ATA***CATATG***CATCATCATCATCATCATCGCTTGGATGCC CCCAGCCAGAT |
| | RV: GCC***GGTACC***TTAAGTGGATGCCTTCACACGTGC |
| TN5-7 | FW: ATA***CATATG***CATCATCATCATCATCATGAGTTGGACACG CCCAAGGAC |
| | RV: GCC***GGATCC***TTATGTTGTGAACTTGGCAGTGATGGTTG |
| TN6-8 | FW: ATA***CATATG***CATCATCATCATCATCATGCCATGGGCTCCCC AAAGGAA |
| | RV: GCC***GGATCC***TTATGTGGTGAAGATGGTCTGGATCAT |
| FBG | FW: ATA***CATATG***CATCATCATCATCATCATATTGGACTCCTGTAC CCCTTCC |
| | RV: GCC***GGATCC***TTATGCCCGTTTGCGCCTGCCT TCAA |

All primers above are written 5' to 3'. Flag sequences are in bold, His tags (CATCATCATCATCATCAT) are underlined, and restriction enzyme cleavage sites (CATATG = Nde1 site, GGATCC = BamH1, GGTACC = Kpn1 site) are in bold italics.

### PCR

PCR amplification was carried out using 10 pmol/µl of each primer, 1µg template, 5µl DMSO, and 1.25 units Pfu Turbo polymerase in a final volume of 25µl. This was added to buffer and dNTPs in Easy mix 50 tubes. The template used for all reactions was cDNA prepared from U87MG human glioma cells using RNA isolated with RNeasy kits. The reaction was cycled 40 times with denaturing, annealing and elongation temperatures of 95°C, 55-65°C (depending on melting temperature (Tm) of primers) and 72°C respectively.

### Cloning

PCR products were ligated into pCR Blunt vectors and sequenced to ensure no errors had been introduced by PCR. Clones were selected that had no errors or silent mutations. Inserts were then ligated into pET32b using Nde1 and BamH1 restriction sites engineered into primers (TN5-7 and TN6-8). Human tenascin-C has internal BamH1 sites within the TA domain (position 494) and TNIII2 (position 2509). TA and TN1-8 were therefore cloned using the Nde1 site in the FW primer and the Kpn1 site in the cloning site of pCRBIunt. Human tenascin-C contains no internal Kpn1 sites. TN1-5, TN1-3 and TN3-5 were cloned using Nde1 and Kpn1 sites in the primers. FBG contains an internal Nde1 site (position 6439) and was therefore cloned using a two step ligation of Nde1 and BamH1 digestion, followed by Nde1 digestion. (Positions refer to sites within the full length nucleotide sequence of tenascin-C, given in figure 14)

### Bacterial growth, induction and lysis

The plasmids were transformed into BL21 (DE3) Rosetta cells, cultured in 3L of Luria-Bertani medium containing 50 µg/ml carbenicillin and induced with 1 mM isopropyl-β-D-thiogalactopyranoside. After 3 hours, the cells were harvested by centrifugation at 4,000 rpm for 20 min, washed twice with ice-cold wash buffer (50 mM Tris-HCI, pH 8.0, 100 mM NaCl, and 1 mM EDTA), and lysed with a French press. Inclusion bodies were collected by centrifugation at 12,000 rpm for 20 min at 4 °C. With the exception of TA and FBG the proteins were located entirely in the supernatant. Recombinant TA and FBG proteins were extracted from inclusion bodies with 6 M guanidine hydrochloride, 50 mM Tris-HCI, pH 8.0, and 10 mM β-mercaptoethanol at room temperature with constant stirring for 2 hours.

### Purification of bacterial proteins

The solution containing recombinant protein was applied to a Ni²⁺-NTA-agarose column and washed with 50 mM Tris-HCI, pH 8.0 containing 20 mM imidazole. The column was subsequently washed with 50 mM Tris-HCI, pH 8.0 and the protein was eluted with 50 mM Tris-HCI, pH 8.0 containing 60 mM imidazole. For TA and FBG each washing and elution buffer contained 6 M guanidine hydrochloride. Following Ni chromatography TA and FBG required no subsequent purification. TN1-3 and TN6-8 were further purified by anion exchange chromatography using a HiTrap Q column, TN1-5, TN3-5 and TN5-7 by cation exchange chromatography using a HiTrap S column, and TN1-8 using a HiTrap S column followed by gel filtration using a Sephacryl S500 HR column.

### Refolding of insoluble proteins

TA and FBG were refolded by diluting to 20 µg/ml with 50 mM Tris-HCI, pH 8.0 containing 6 M guanidine hydrochloride and then treating with 20 mM cystamine with stirring for 16 hours at 4 °C. The solution was then dialyzed twice against 15 volumes of 50 mM Tris-HCI, pH 8.0 containing 150 mM NaCl, 10 mM CaCl₂, 5 mM β-mercaptoethanol, and 1 mM 2-hydroxyethyl disulfide for 24 hours at 4 °C, twice against 20 mM Tris-HCI, pH 8.0 for 8 hours at 4 °C and then centrifuged at 12,000 rpm for 30 min at 4 °C. Refolding was assessed by size shifts using SDS PAGE under reducing and non reducing conditions. Protein activity was confirmed by TA domain polymerization and FBG binding to heparin sepharose columns.

### Synthesis of EGF-L domain using mammalian cells

Initial attempts to express and purify the EGF-L repeats region using an *E.coli* expression system were unsuccessful. This is most likely to be attributable to difficulty in achieving protein folding due to a total of 91 cysteines in this region. Therefore, the EGF-like domains of TN-C were expressed using HEK293 cells.

Two PCR reactions were carried out. The first PCR product consisted of a restriction enzyme *Kpn*I site, a Kozak sequence followed by the TN-C signal sequence. The second PCR product consisted of a FLAG peptide, the EGF-like domain sequence, followed by a histidine tag and a BamH1 restriction enzyme sequence.

The two PCR products were ligated together as described by Ho (1989). PCR reactions were carried out as described above. The entire construct was cloned into the PCR blunt vector and sequenced. It was then subcloned into the pCEP4 vector. The DNA was transfected into HEK293 cells using Fugene and cells were selected for hygromycin resistance (200 µg/ml) in Dulbecco's modified Eagle's medium (DMEM) containing 10% (v/v) fetal calf serum, penicillin (100 units/ml) and streptomycin (100 units/ml). 2 litres conditioned medium (collected after cells have been cultured in medium) from stably transfected cells was collected and pooled. The pooled conditioned medium (2 litres) was centrifuged at 3000 rpm to separate cell debris from the medium.

The medium was then applied to an anti-FLAG column. Material was collected in 50 ml fractions for the flow-through. The column was washed with 10 column volumes of 1M NaCl, 50 mM Tris-HCI, pH 7.5 and then washed with 10 column volumes of 60% isopropanol to ensure removal of LPS. The column was then washed with 50 mM Tris-HCI buffer, pH 7.5 and finally the protein was eluted using 200 µg/ml FLAG peptide in 50 mM Tris-HCI buffer, pH 7.5.

### Analysis of protein purity

Each protein was dialysed against 1000 volumes of 150 mM NaCl and 50mM Tris pH 7.5. Protein purity was analyzed by SDSPAGE under reducing conditions. To do this 1 µg of each purified recombinant protein was run on a 4-12% Bis-Tris gradient gel and the gel was subsequently silver stained to demonstrate a single band (figure 10). Western blotting analyses were also carried out. Proteins separated by SDS-PAGE were electrotransferred to polyvinylidene difluoride membranes. The membranes were blocked with 5% BSA in Tris-buffered saline and then incubated with primary antibodies recognizing FLAG M2 (1:2000 dilution)(EGF-L) or tetra-his antibodies (1:2000)(all other proteins). The blot was then incubated with secondary antibody conjugated to alkaline phosphatase and the protein bands visualized using Western Blue stabilized substrate whereby the gels show a single specific band recognised by each antibody at the expected Mw (not shown)

### Example 3 - Animal models

### Zymosan-induced arthritis

Zymosan-induced arthritis (ZIA) was induced in tenascin-C deficient and wild type mice by injection of zymosan (*Saccharomyces cerevisiae*), as described in Keystone (1977). Zymosan was prepared by dissolving 15 mg of zymosan in 1ml of sterile PBS. The solution was boiled twice and sonicated. Mice were anesthetized by intraperitoneal injection of 150 µl of Hypnorm diluted 1:10 in sterile water, then injected with zymosan (10 µl) into the right footpad (d=0).

Control mice received an injection of 10 µl PBS alone or were not injected. For macroscopic assessment of arthritis, the thickness of each hind paw was measured daily with microcalipers (Kroeplin, Schluchlem, Germany) and the diameter expressed as an average for each inflamed hind paw per mouse.

Following completion of the experiment (day=4), mice were euthanized and hind paws fixed in 10% (v/v) buffered formalin, decalcified with 10% EDTA and processed to paraffin.

### Antigen-induced arthritis

Antigen-induced arthritis (AIA) was induced in tenascin-C-deficient and wild-type mice as described previously by Brackertz (1977). Briefly, at day 0 mice were anesthetized by intraperitoneal injection of 150 µl of Hypnorm diluted 1:10 in sterile water, then immunized with 200 µg of methylated BSA. mBSA was emulsified in 0.2 ml of Freund's complete adjuvant and injected intra-dermally at the base of the tail.

At day 7, arthritis was induced by intra-articular injection of mBSA (100 µg in 10 µl of sterile PBS) into the right knee joint using a sterile 33-gauge microcannula. Control mice received an injection of 10 l PBS alone or were not injected.

On day 14, mice were euthanized, the knee joints were excised and fixed in 10% (volume/volume) buffered formalin, decalcified, with 10% EDTA and processed to paraffin.

### Injection of FBG

Wild type mice were anesthetized by intraperitoneal injection of 150 µl of Hypnorm diluted 1:10 in sterile water and then injected with 100 ng, 1 or 3 µg FBG in 10 µl of sterile PBS into the right knee joint using a sterile 33-gauge microcannula. Control mice received an injection of 10 µl PBS alone or were not injected.

On days 3 and 7, mice were euthanized, the knee joints were excised and fixed in 10% (volume/volume) buffered formalin, decalcified, with 10% EDTA and processed to paraffin.

### Histology of knee joints

Coronal tissue sections (4 µm) were cut at 7 depths throughout the joint; 80 µm apart and stained with hematoxylin and eosin or Safranin-O to assess joint pathology. Histopathologic changes were scored using the following parameters as described in Van Lent (2006).

Inflammation (the influx of inflammatory cells into synovium (infiltrate) and the joint cavity (exudates), was graded using an arbitrary scale from 0 (no inflammation) to 3 (severe inflammation). Chondrocyte death was determined as the percentage of cartilage area containing empty lacunae in relation to the total area. Cartilage surface erosion was determined as the amount of cartilage lost in relation to the total cartilage area. Bone destruction was determined in 10 different areas of the total knee joint section. Destruction was graded on a scale of 0 (no damage) to 3 (complete loss of bone structure). Histological analysis was performed by an investigator who was blinded to the experimental groups. The mean score for each animal in an experimental group was calculated by averaging the histopathologic scores in at least 5 section depths per joint.

### Results

### Zymosan induced joint inflammation is not sustained in tenascin-C deficient mice

Zymosan injection into the footpad was used to induce acute synovitis in mice. Wild type mice exhibited rapid paw swelling reaching maximal paw diameter by 24 hours (2.56mm, an increase of 62% of the starting paw diameter). This was maintained for a further 24 hours. After 2 days paw diameter decreased but paws remained swollen by 4 days (2.08mm, an increase of 32%) (figure 1a). tenascin-C deficient mice exhibited a similar degree of paw swelling to wild type mice 24 hours post injection (2.41mm, an increase of 57% of starting paw diameter). However, swelling in the tenascin-C null mice subsided faster than in the wild type mice; paw diameter was significantly reduced at 2 days and had declined to 1.7mm (an increase of only 11%) by 4 days (figure 1a). By day 4 post injection the paws of wild type mice were still visibly swollen and red, whereas the paws of tenascin-C null mice were not visibly swollen or red and resembled non-injected paws (Figure 9).

This difference was reflected histologically at 4 days. The synovia of wild type mice were significantly inflamed and exhibited cellular infiltration and cartilage proteoglycan loss was observed (Figure 1b, c). In contrast, the synovium of tenascin-C deficient mice exhibited no synovitis, cellular infiltrate or cartilage proteoglycan loss (figure 1d, e) and resembled the joints of sham injected and non injected mice (not shown). Quantification of joint inflammation revealed whilst there was little exudate (cellular mass in the joint cavity) in either wild type or tenascin-C null mice, levels of infiltrate (cellular mass in the synovial layer) were significantly reduced in tenascin-C null mice (figure 1f). No erosion of cartilage or bone occurred in mice of either genotype (not shown), however a low level of chondrocyte death occurred in wild type mice, that was not observed in tenascin-C null mice (figure 1g). Thus tenascin-C expression appears to promote the maintenance of acute inflammation.

### Tenascin-C null mice are protected from persistent inflammation and structural damage during antigen induced arthritis

To determine whether tenascin-C also contributes to more destructive inflammatory joint disease, erosive arthritis was induced by intra-articular injection of mBSA into the knee joint following immunization with mBSA. This model involves both cellular and humoral immune responses and induces pathological changes similar to human RA (Brackertz (1977)). Injection of mBSA induced a similar inflammatory response in both tenascin-C null and wild type mice. Cell infiltration and synovial thickening is apparent by 24 hours in mice of both genotypes (figure 2c-f, h, i) compared to sham injected (figure 2a, b, g) or non injected (not shown) mice.

However, this does not persist in tenascin-C null mice as it does the wild type mice. By 3 days post injection wild type mice exhibit increased inflammation of the meniscus and capsule, synovial hyperplasia, cells and fibrin deposits in the joint space, pannus formation and localized cartilage proteoglycan loss (figure 3a, b, f). In contrast, by 3 days in tenascin-C null mice inflammation is limited to the capsule, synovial inflammation has subsided and there are no fibrin/cell aggregates present in the joint space, no pannus formation and no cartilage proteoglycan loss (figure 3c, d, e).

By 7 days wild type mice exhibited persistent inflammatory cell infiltration and joint space exudate, extensive synovitis and pannus formation and destruction of articular cartilage and bone erosion (figure 4a, b). Sham injected knees and knees from mice that had undergone no injection were healthy and exhibited no inflammation or joint destruction (not shown). tenascin-C deficient mice also had healthy joints that exhibited only mild inflammatory cell infiltration, with no joint space exudate, synovitis, pannus formation, destruction of articular cartilage or bone erosion (figure 4c, d). Joints from tenascin-C deficient mice that had been sham injected and or that had undergone no injection were also healthy (not shown).

These histological data are reflected upon scoring of joint disease as described in materials and methods. Levels of cellular infiltrate and exudate observed in both wild type mice and tenascin-C null mice 24 hours post injection were not significantly different. However, whilst cellular mass continued to increase in wild type mice over time, this response was attenuated in tenascin-C null mice and cell numbers in the joint decreased over time (figure 4e). Increasingly high levels of chondrocyte death occurred in the cartilage of wild type mice over time, but no significant death was observed in tenascin-C null mice (figure 4f). No cartilage surface erosion and bone erosion was evident in wild type mice at 24 hours or 3 days (not shown) but significant tissue destruction had occurred by 7 days. In contrast tenascin-C null mice exhibited no tissue destruction at 24 hours, 3 days (not shown) or 7 days (figure 4f). These data indicate that whilst the initiation of joint inflammation (cell influx into the synovium and joint space) is unaffected in tenascin-C null mice, unlike in wild type mice disease does not progress to tissue destruction and cell death. These results demonstrate that expression of tenascin-C is required for persistent synovial inflammation and joint destruction in this model.

### Example 4 - Cell culture

### Patient Specimens

Human monocytes were isolated from peripheral blood (London Blood Bank) and macrophages were derived from monocytes after differentiation for 4 days with 100 ng/ml of M-CSF as previously described (Foxwell (1998)).

RA membrane cells (representing a mixed population of all synovial cell types) were isolated from synovial membranes obtained from patients undergoing joint replacement surgery as previously described (Brennan(1989)). RA synovial fibroblasts were isolated from the mixed population of RA membrane cells as previously described (Brennan(1989)). The study was approved by the local Trust ethics committee (Riverside NHS Research Committee), and waste tissue (synovium after joint replacement surgery) was obtained only after receiving signed informed consent from the patient and anonymyzing the tissue to protect patient identity.

Immediately after isolation, RA membrane cells and macrophages were cultured at 1x10⁵ cells/well in RPMI 1640 containing 10% (v/v) FBS and 100 U/ml (Units/ml) penicillin/streptomycin in 96-well tissue culture plates for 24 hours before stimulation. Synovial fibroblasts (used only at either passage number 2 or 3) were cultured at 1x10⁴ cells/well in DMEM containing 10% (v/v) FBS and 100 U/ml penicillin/streptomycin in 96-well tissue culture plates for 24 hours before stimulation.

### Mouse embryonic fibroblasts (MEFs) and bone marrow derived macrophages (BMDMs)

MEFs express high levels of mRNA of all 9 murine TLRs and are specifically and highly responsive to TLR ligand activation. MEFs from mice with targeted deletions of TLR2, TLR4 and MyD88 demonstrate profound defects in their IL-6 response to specific ligands (Kurt-Jones (2004)). MEFs were isolated from d13 embryos harvested from age-matched, pregnant female wild type, TLR2, TLR4 and null mice (as described in Todaro (1963)). Fibroblasts were cultured at 2x10⁴ cells/well in DMEM containing 10% (v/v) FBS and 100 U/ml penicillin/streptomycin in 96-well tissue culture plates for 24 hours before stimulation.

BMDMs were derived by aspirating the femurs of age matched female wild type, TLR2 and TLR4 null mice as described in Butler (1999)) and culturing the cells for 7 days in DMEM, 20% (v/v) FBS, 10ml/L (v/v) antibiotic-antimycotic solution PSA, 50µM β-Mercaptoethanol and 10ng/ml M-CSF. Macrophages were then cultured at 1x10⁵ cells/well in DMEM, 20% (v/v) FBS, 10ml/L (v/v) antibiotic-antimycotic solution PSA, 50µM β-Mercaptoethanol in 96-well tissue culture plates for 24 hours before stimulation.

### HEK293 cell lines

HEK293 cell lines expressing TLR2 and TLR4/CD14/MD-2 were cultured at 1x10⁴ cells/well in DMEM containing 10% (v/v) FBS and 10 µg/ml blasticidin in 96-well tissue culture plates for 24 hours before stimulation.

### Cell stimulation and assessment of cytokine synthesis

Cells were incubated for 24 hours at 37°C with the indicated doses of tenascin-C and recombinant tenascin-C fragments (1.0 µM - 1.0 nM). Cells were also stimulated where indicated with LPS (1 ng/ml for human macrophages, 10ng/ml for human fibroblasts, RA membrane cells and HEKs, 100ng/ml for MEFS and BMDMs and 10ng/ml for HEKS), PAM3 (10 ng/ml for human macrophages, human fibroblasts, and HEKs, 100ng/ml for MEFs and BMDMs), murine IL-1 (5ng/ml for MEFS) and murine TNF-α (100ng/ml for MEFS). Unless specifically stated otherwise rough LPS was used for *in vitro* studies.

For adenoviral gene transfer experiments, human RA synovial fibroblasts were incubated with adenoviral vectors at a multiplicity of infection of 100, washed after 2 hours, cultured in complete medium for 24 hours, then stimulated for 24 hours, after which time supernatants were collected.

Where stated, cells were pre-incubated with 10µg/ml anti-CD14 antibody, 10µg/ml IL1 receptor antagonist, 10µg/ml anti-TLR2 antibody, 25µg/ml anti-TLR4 antibody, 10 or 25µg/ml isotype control antibody, 25µg/ml polymyxin B, or 1µg/ml msbB LPS, for 30 minutes at 37°C before stimulation. Where stated, recombinant tenascin-C and FBG, and LPS were boiled for 15 minutes before addition to cells

In all cases, viability of the cells was not significantly affected throughout the experimental time period when examined by the MTT cell viability assay (Sigma, Poole, UK).

Supernatants were subsequently examined for the presence of the cytokines TNF-α, IL-6, and IL-8 by enzyme-linked immunosorbent assay (ELISA) according to the manufacturer's instructions. Absorbance was read on a spectrophotometric ELISA plate reader (Labsystems Multiscan Biochromic, Vantaa, Finland) and analyzed using the Ascent software program (Thermo Labsystems, Altrincham, UK).

### Results

### Tenascin-C induces TNF-α, IL-6 and IL-8 synthesis in primary human RA synovial fibroblasts and macrophages

We next investigated whether tenascin-C might activate the innate immune response. tenascin-C was used to stimulate primary human macrophages and RA synovial fibroblasts and the production of the pro-inflammatory cytokines TNF-α, IL-6 and IL-8 examined. The bacterial cell wall component LPS was used as a positive control. tenascin-C induced a cell type specific cytokine profile which was significantly different from LPS. It dose dependently stimulated the production of TNF-α, IL-6 and IL-8 in human macrophages (figure 5a). However, tenascin-C only induced IL-6 synthesis in synovial fibroblasts, whereas LPS induced both IL-6 and IL-8 (figure 5b). Neither LPS nor tenascin-C induced TNF-α synthesis in fibroblasts (data not shown). tenascin-C stimulation of IL-6 (Figure 5c), IL-8 and TNF-α by human macrophages and IL-6 by synovial fibroblasts (not shown) was heat sensitive and unaffected by the LPS inhibitor, polymyxin B. Together these results provide strong evidence that cytokine induction by tenascin-C is not due to LPS contamination.

### The fibrinogen-like globe (FBG) mediates tenascin-C activation of cells.

Tenascin-C is a large hexameric molecule, each domain of which binds to different cell surface receptors (reviewed in Orend (2005)). Understanding the mechanism of action of tenascin-C will require identification of which domain(s) are critical for promoting cytokine production. We synthesized recombinant proteins comprising different domains of the molecule (figure 10). Each domain was made in *E.coli,* purified (figure 11), and found to contain <10 pg/ml LPS by subjecting neat protein to the *Limulus amaebocyte* lysate assay. Only one domain of tenascin-C was active. The fibrinogen-like globe (FBG) stimulated TNF-α synthesis in human macrophages (Figure 6a), IL-6 and IL-8 synthesis in human macrophages (not shown) and IL-6 in RA synovial fibroblasts (not shown) to an equal extent to full-length tenascin-C. Like full-length tenascin-C, FBG did not induce IL-8 synthesis in RA synovial fibroblasts where LPS did (data not shown). FBG induced cytokine synthesis was also heat sensitive and unaffected by polymyxin B (data not shown).

### The FBG domain of tenascin-C induces cytokine production in human RA synovium and joint inflammation in mice.

We investigated whether FBG could promote expression of inflammatory cytokines in synovial membranes from RA patients. This tissue model of RA (comprising a mixed population of all synovial cell types) spontaneously produces high levels of IL-6, IL-8 and TNF-α (Brennan (1989)) (figure 6b). FBG further enhanced synthesis of all these cytokines (figure 6b). To determine whether FBG could induce inflammation *in vivo,* wild type mice were injected intra-articularly with FBG. We observed a transient and dose dependent stimulation of joint inflammation. No inflammation or proteoglycan loss occurred in non-injected mice or in mice injected with PBS (figure 6c-e) or 100ng FBG (data not shown). In mice injected with 1 µg FBG inflammatory cell infiltration (figure 6f), mild synovitis, pannus formation (figure 6g) and proteoglycan loss (figure 6h) was observed. A similar response was seen in mice injected with 3 µg FBG (data not shown). Upon histological quantification, high levels of cellular infiltrate and exudate and chondrocyte death were observed in mice injected with FBG, together with a modest amount of cartilage surface erosion and bone damage (figure 6i).

### FBG mediated cytokine synthesis is dependent on Myd88

Many DAMPs, including fibrinogen (Smiley (2001)), have been shown to stimulate the innate immune response by activation of TLRs. Therefore, we investigated whether TLRs might also mediate tenascin-C induced cytokine production. Myeloid differentiation factor 88 (MyD88) is required for signalling by all TLRs, except TLR3 (O'Neill (2008)). Infection of synovial fibroblasts with adenovirus expressing dominant negative MyD88, but not GFP control virus, abolished FBG induction of IL-6 (figure 7a). These data suggest that FBG induced inflammation is dependent on functional MyD88. This effect of FBG did not appear to be mediated by IL-1 as addition of IL-1 receptor antagonist did not inhibit induction of cytokines (data not shown). To confirm that FBG action is MyD88 dependent we demonstrated that FBG does not stimulate cytokine synthesis in embryonic fibroblasts isolated from mice with targeted deletions in the MyD88 gene. The TLR2 ligand PAM3, TLR4 ligand LPS and IL-1 all signal via MyD88. Stimulation with these was also abolished in MEFs from deficient mice. However, TNF-α, which does not signal via MyD88, was unaffected (figure 7b). Re-transfection of wild type MyD88 restored the responsiveness of these cells to FBG, PAM3, LPS and IL-1 (data not shown).

### FBG signals via TLR4

TLRs exhibit specificity for endogenous ligands; proteins are recognised by one or both of TLR2 and 4 (reviewed in O'Neill (2008)). Neutralising antibodies to TLR4 inhibited both FBG and LPS induced IL-6, IL-8 and TNF-α synthesis in human macrophages and IL-6 synthesis in RA synovial fibroblasts but had no effect on the function of the TLR2 ligand, PAM3. Antibodies to TLR2 inhibited PAM3 mediated cytokine synthesis but had no effect on LPS or FBG induced cytokine synthesis. Isotype matched controls had no effect on cytokine synthesis induced by any ligand (TNF-α synthesis by human macrophages is shown in figure 8a). To confirm that FBG action is TLR4 dependent we demonstrated that FBG does not stimulate cytokine synthesis in embryonic fibroblasts or macrophages isolated from mice with targeted deletions in the TLR4 gene. FBG mediated cytokine synthesis was unaffected in embryonic fibroblasts or macrophages isolated from mice with targeted deletions in the TLR2 gene. Cells isolated from TLR2 deficient mice were unresponsive to PAM3 but responsive to LPS and IL-1. Cells isolated from TLR4 deficient mice were unresponsive to LPS but did respond to PAM3 and IL-1 (figure 8b, c). In addition, expression of TLR4 was required for the arthritogenic action of FBG in vivo; FBG was able to induce joint inflammation in TLR2 null mice but not in TLR4 null mice (figure 12).

### Different co-receptor requirements for FBG and LPS

LPS signalling via TLR4 is mediated by a receptor complex including the soluble protein MD-2 and GPI-linked cell surface or soluble CD14 (reviewed in Fitzgerald (2004)). We next examined whether CD14 and MD-2 are required for FBG activation of TLR4. As a positive control here we examined the activity of smooth glycosylated LPS which requires both MD-2 and CD14 (Jiang (2005)). LPS mediated IL-6, IL-8 and TNF-α synthesis by human macrophages and IL-6 synthesis by RA synovial fibroblasts was inhibited by anti-CD14 antibodies and an antagonistic LPS derived from the msbB mutant E.coli which competes for LPS binding to MD-2 (Coats(2007)). Conversely, both PAM3, which does not require these co-receptors for activation of TLR2, and FBG-mediated cytokine synthesis was unaffected by anti CD14 antibodies or msbB mutant LPS (figure 8d shows TNF-α synthesis by human macrophages). These data suggest that neither CD14 nor MD-2 is required for FBG mediated cytokine synthesis. Therefore, whilst LPS and FBG both signal via activation of TLR4, they may have different co-receptor requirements.

### Example 5 - Inhibition of Tenascin-C action and synthesis in human tissue

This example studies the effect of (1) prevention ofn the pro-inflammatory action of tenascin-C and (2) inhibition of tenascin-C expression in the human RA synovium.

### Methods

### Peptide synthesis

Nine overlapping peptides comprising the entire FBG domain (table 2) were synthesized by Biogenes, Germany. Peptides were cleaved at room temperature (cleavage mixture: 90% trifluoroacetate, 5% thioanisol, 3% ethanedithiol, 2% anisole), purified by reverse phase high performance liquid chromatography, and characterized by MALDI TOF mass spectral analysis. The purity of the peptides was >85% as determined high performance liquid chromatography.

The facility was unable to synthesize peptide 7, presumably due to the formation of secondary structure that prevented elongation of the peptide chain (as previously reported (LaFleur (1997)).

**Table 2. Overlapping peptides that span the entire FBG domain of human tenascin-C**

| **Peptide #** | **Amino acid sequence** |
|---|---|
| 1 | TIGLLYPFPKDCSQAMLNGDTTSGLYTIYL |
| 2 | YTIYLNGDKAEALEVFCDMTSDGGGWIVFL |
| 3 | WIVFLRRKNGRENFYQNWKAYAAGFGDRRE |
| 4 | GDRREEFWLGLDNLNKITAQGQYELRVD |
| 5 | ELRVDLRDHGETAFAVYDKFSVGDAKTRYK |
| 6 | KTRYKLKVEGYSGTAGDSMAYHNGRSFST |
| 7 | RSFSTFDKDTDSAITNCALSYKGAFWYRN |
| 8 | WYRNCHRVNLMGRYGDNNHSQGVNWFHWKG |
| 9 | FHWKGHEHSIQFAEMKLRPSNFRNLEGRRKRA |

### Patient specimens and cell culture

RA membrane cells (representing a mixed population of all synovial cell types) were isolated from synovial membranes obtained from patients undergoing joint replacement surgery (Brennan (1989)). Synovial membrane tissue was digested in RPMI 1640 (GIBCO) containing 5% fetal calf serum (FCS) (GIBCO), 5 mg/ml collagenase type IV (Sigma) and 0 15 mg/ml DNAse type I (Sigma) and incubated at 37°C for 2 h.

After incubation the tissue was pipetted through a nylon mesh into a sterile beaker. The cells were then washed three times in complete medium (RPMI 1640 supplemented with 10% FCS). RA synovial fibroblasts were isolated from the mixed population of RA membrane cells by selection in DMEM (Bio-Whittaker) supplemented with 10% FBS, 1 µM glutamine, 100 U/ml penicillin, and streptomycin. Human monocytes were isolated from peripheral blood (London Blood Bank) and macrophages were derived from monocytes after differentiation for 4 days with 100 ng/ml of M-CSF.

The study was approved by the local Trust ethics committee, and waste tissue (synovium after joint replacement surgery) was obtained only after receiving signed informed consent from the patient and anonymyzing the tissue to protect patient identity.

### Cell stimulation and assessment of cytokine synthesis

Immediately after isolation, RA membrane cells were cultured at 1x10⁵ cells/well in RPMI 1640 containing 10% (v/v) FBS and 100 U/ml penicillin/streptomycin in 96-well tissue culture plates. Cells were incubated for 24h at 37°C with no addition, buffer control (PBS, 1% BSA, 0.01% NaN₃), or with 25 µm, 100 µM or 250 µM of each FBG spanning peptide.

Synovial fibroblasts (used only at either passage number 2 or 3) were seeded at a concentration of 5 × 10⁴ cells in a 3.5-cm dish. siRNA was transfected at a final concentration of 10 nM using Lipofectamine 2000 (Invitrogen) for 4 h in serum-free OptiMEM I. Two different siRNAs against human tenascin-C were used (s7069 and s229491) (Applied Biosystems).

siRNA sequences of s7069 are: (sense 5' CGCGAGAACUUCUACCAAAtt 3', antisense 5' UUUGGUAGAAGUUCUCGCGtc 3') and of s229491 are (5' GGAAUAUGAAUAAAGAAGAtt 3', antisense 5' UCUUCUUUAUUCAUAUUCCgg 3'). siRNA against luciferase (Dharmacon) was transfected as a non-targeting control.

Four hours after transfection, medium was changed with pre-equilibrated Dulbecco's modified Eagle's medium containing 10% FBS (v/v) and cells were incubated for a further 48h and 72h. Cells were then stimulated with 10 ng/ml LPS for 24h at 37°C. Tenascin-C mRNA and protein levels were quantitated by PCR and western blotting respectively. Total RNA was extracted from cells using a QiaAmp RNA Blood mini kit (Qiagen, Germany). cDNA was synthesised from equivalent amounts of total RNA using SuperScript® III Reverse Transcriptase (Invitrogen) and 18-mer oligo dTs (Eurofins MWG Operon).

Gene expression was analysed by delta-delta ct methods based on quantitative real-time PCR with TaqMan primer set human tenascin-C (Hs01115663-m1) and human ribosomal protein endogenous control (RPLPO) (4310879E) (Applied Biosystems) in a Corbett Rotor-gene 6000 machine (Corbett Research Ltd). Tenascin-C protein was detected in cell supernatants and cell lysates by by SDS PAGE and western blotting using antibody MAB1908 (Millipore).

Macrophages were cultured at 1x10⁵ cells/well in RPMI 1640 containing 5% (v/v) FBS and 100 U/ml penicillin/streptomycin in 96-well tissue culture plates for 24h before stimulation. Cells were incubated for 24h at 37°C with no addition, 1.0 µM FBG, 1 ng/ml LPS or 1 or 20 µM FBG peptide. Where stated, cells were pre-incubated with 20 µM FBG peptides for 15 min.

The viability of the cells was not significantly affected throughout the experimental time period when examined by the MTT cell viability assay (Sigma, Poole, UK). Supernatants were examined for the presence of the cytokines TNF-α, IL-6, and IL-8 by enzyme-linked immunosorbent assay (ELISA) according to the manufacturer's instructions (R&D systems). Absorbance was read on a spectrophotometric ELISA plate reader (Labsystems Multiscan Biochromic, Vantaa, Finland) and analyzed using the Ascent software program (Thermo Labsystems, Altrincham, UK).

### Statistical Methods

Mean, SD and SEM were calculated using GraphPad (GraphPad Software Inc., San Diego, CA).

### Results

### Blockade of cytokine synthesis in RA membrane cultures by specific FBG peptides

The approach of peptide inhibition has been used successfully to pinpoint the αvβ3 integrin binding site in the FBG domain of tenascin-C and to prevent cell adhesion in response to this domain of tenascin-C (Lafleur (1997) and Yokoyama (2000)).

We synthesized a series of 8 overlapping peptides of -30 amino acids that span the entire sequence of FBG (Table 2). Peptides were tested for the ability to block spontaneous cytokine synthesis in RA synovial membrane cultures. TNF and IL8 synthesis was inhibited by peptides 3 and 8, but not by any other peptide (TNF shown in figure 15). Peptides 3 and 8 dose dependently inhibited cytokine synthesis with the highest concentrations achieving 95% and 56% inhibition respectively (figure 16). Whilst peptide 5 had no effect on TNF synthesis, it dose dependently blocked IL8 synthesis in RA membrane cells with a maximal inhibition of 81% (figure 16).

To map the active domain within FBG responsible for inducing cytokine production we stimulated primary human macrophages with each FBG peptide. Peptides 1, 5 and 6 all induced cytokine synthesis in a dose dependent manner. (figure 17).

To determine if any peptide could block FBG induced cytokine synthesis in human macrophages, cells were pre-incubated with each FBG peptide before stimulation with either whole FBG or LPS. Peptide 5 specifically blocked FBG mediated cytokine synthesis, whilst peptide 8 blocked cytokine synthesis in response to both LPS and FBG (figure 18).

Peptide 8 therefore non-specifically blocks cytokine production induced by any stimuli. This domain is the integrin binding domain of FBG that mediates cell adhesion and thus may be acting to prevent cell attachment to tissue culture plates. Peptide 5 specifically blocks FBG-induced cytokine synthesis suggesting that targeting this domain may be useful in preventing tenascin-C induced inflammation.

### Silencing tenascin-C gene expression inhibits cytokine synthesis in RA synovial fibroblasts

Examination of the effect of inhibiting tenascin-C expression in the human RA synovium has identified synovial fibroblasts as the major source of tenascin-C in RA (figure 1C) (in Goh 2010).

siRNA mediated knockdown of tenascin-C expression in these cells has been shown with a maximal efficiency between 94-96% (figure 19). In cells transfected with tenascin-C siRNA, both the basal level of cytokine synthesis and LPS induced cytokine production was inhibited by 38% and 44% respectively compared to control cells (figure 19)

These data reveal that silencing tenascin-C in RA synovial fibroblasts reduces the synthesis of pro-inflammatory cytokines and suggest that ablation of tenascin-C expression is a viable strategy to inhibit inflammation in the synovium.

This work has established that blocking tenascin-C activity (with peptides) and tenascin-C expression (with siRNA) reduces inflammatory cytokine synthesis in human RA synovia. These data shows that tenascin-C blockade is of potential clinical benefit in treating RA and other inflammatory diseases.

### References

1. Smolen, J.S. & Maini, R.N. Interleukin-6: a new therapeutic target. Arthritis Res Ther 8 Suppl 2, S5 (2006).
2. Williams, R.O., Paleolog, E. & Feldmann, M. Cytokine inhibitors in rheumatoid arthritis and other autoimmune diseases. Curr Opin Pharmacol 7, 412-417 (2007).
3. Brentano, F., Kyburz, D., Schorr, O., Gay, R. & Gay, S. The role of Toll-like receptor signalling in the pathogenesis of arthritis. Cell Immunol 233, 90-96 (2005).
4. O'Neill, L.A. Primer: Toll-like receptor signaling pathways-what do rheumatologists need to know? Nat Clin Pract Rheumatol (2008).
5. Matzinger, P. The danger model: a renewed sense of self. Science 296, 301-305 (2002).
6. Bianchi, M.E. DAMPs, PAMPs and alarmins: all we need to know about danger. J Leukoc Biol 81, 1-5 (2007).
7. Gordon, S. Pattern recognition receptors: doubling up for the innate immune response. Cell 111, 927-930 (2002).
8. Medzhitov, R. & Janeway, C.A., Jr. Decoding the patterns of self and nonself by the innate immune system. Science 296, 298-300 (2002).
9. Radstake, T.R., et al. Expression of toll-like receptors 2 and 4 in rheumatoid synovial tissue and regulation by proinflammatory cytokines interleukin-12 and interleukin-18 via interferon-gamma. Arthritis Rheum 50, 3856-3865 (2004).
10. Roelofs, M.F., et al. The expression of toll-like receptors 3 and 7 in rheumatoid arthritis synovium is increased and costimulation of toll-like receptors 3, 4, and 7/8 results in synergistic cytokine production by dendritic cells. Arthritis Rheum 52, 2313-2322 (2005).
11. Sacre, S.M., et al. The Toll-like receptor adaptor proteins MyD88 and Mal/TIRAP contribute to the inflammatory and destructive processes in a human model of rheumatoid arthritis. Am J Pathol 170, 518-525 (2007).
12. Choe, J.Y., Crain, B., Wu, S.R. & Corr, M. Interleukin 1 receptor dependence of serum transferred arthritis can be circumvented by toll-like receptor 4 signaling. J Exp Med 197, 537-542 (2003).
13. Lee, E.K., Kang, S.M., Paik, D.J., Kim, J.M. & Youn, J. Essential roles of Toll-like receptor-4 signaling in arthritis induced by type II collagen antibody and LPS. Int Immunol 17, 325-333 (2005).
14. Abdollahi-Roodsaz, S., et al. Inhibition of Toll-like receptor 4 breaks the inflammatory loop in autoimmune destructive arthritis. Arthritis Rheum 56, 2957-2967 (2007).
15. Vanags, D., et al. Therapeutic efficacy and safety of chaperonin 10 in patients with rheumatoid arthritis: a double-blind randomised trial. Lancet 368, 855-863 (2006).
16. Chiquet-Ehrismann, R. & Chiquet, M. Tenascins: regulation and putative functions during pathological stress. J Pathol 200, 488-499 (2003).
17. Cutolo, M., Picasso, M., Ponassi, M., Sun, M.Z. & Balza, E. Tenascin and fibronectin distribution in human normal and pathological synovium. J Rheumatol 19, 1439-1447 (1992).
18. McCachren, S.S. & Lightner, V.A. Expression of human tenascin in synovitis and its regulation by interleukin-1. Arthritis Rheum 35, 1185-1196 (1992).
19. Salter, D.M. Tenascin is increased in cartilage and synovium from arthritic knees. Br J Rheumatol 32, 780-786 (1993).
20. Chevalier, X., Groult, N., Larget-Piet, B., Zardi, L. & Hornebeck, W. Tenascin distribution in articular cartilage from normal subjects and from patients with osteoarthritis and rheumatoid arthritis. Arthritis Rheum 37, 1013-1022 (1994).
21. Hasegawa, M., et al. Expression of large tenascin-C splice variants in synovial fluid of patients with rheumatoid arthritis. J Orthop Res 25, 563-568 (2007).
22. Orend, G. Potential oncogenic action of tenascin-C in tumorigenesis. Int J Biochem Cell Biol 37, 1066-1083 (2005).
23. Brackertz, D., Mitchell, G.F. & Mackay, I.R. Antigen-induced arthritis in mice. I. Induction of arthritis in various strains of mice. Arthritis Rheum 20, 841-850 (1977).
24. Brennan, F.M., Chantry, D., Jackson, A., Maini, R. & Feldmann, M. Inhibitory effect of TNF alpha antibodies on synovial cell interleukin-1 production in rheumatoid arthritis. Lancet 2, 244-247 (1989).
25. Smiley, S.T., King, J.A. & Hancock, W.W. Fibrinogen stimulates macrophage chemokine secretion through toll-like receptor 4. J Immunol 167, 2887-2894 (2001).
26. Fitzgerald, K.A., Rowe, D.C. & Golenbock, D.T. Endotoxin recognition and signal transduction by the TLR4/MD2-complex. Microbes Infect 6, 1361-1367 (2004).
27. Jiang, Z., et al. CD14 is required for MyD88-independent LPS signaling. Nat Immunol 6, 565-570 (2005).
28. Coats, S.R., Do, C.T., Karimi-Naser, L.M., Braham, P.H. & Darveau, R.P. Antagonistic lipopolysaccharides block E. coli lipopolysaccharide function at human TLR4 via interaction with the human MD-2 lipopolysaccharide binding site. Cell Microbiol 9, 1191-1202 (2007).
29. Siri, A., et al. Human tenascin: primary structure, pre-mRNA splicing patterns and localization of the epitopes recognized by two monoclonal antibodies. Nucleic Acids Res 19, 525-531 (1991).
30. Gondokaryono, S.P., et al. The extra domain A of fibronectin stimulates murine mast cells via toll-like receptor 4. J Leukoc Biol 82, 657-665 (2007).
31. Taylor, K.R., et al. Recognition of hyaluronan released in sterile injury involves a unique receptor complex dependent on Toll-like receptor 4, CD44, and MD-2. J Biol Chem 282, 18265-18275 (2007).
32. Kim, H.M., et al. Crystal structure of the TLR4-MD-2 complex with bound endotoxin antagonist Eritoran. Cell 130, 906-917 (2007).
33. Schaefer, L., et al. The matrix component biglycan is proinflammatory and signals through Toll-like receptors 4 and 2 in macrophages. J Clin Invest 115, 2223-2233 (2005).
34. Foell, D., Wittkowski, H. & Roth, J. Mechanisms of disease: a 'DAMP' view of inflammatory arthritis. Nat Clin Pract Rheumatol 3, 382-390 (2007).
35. Taniguchi, N., et al. High mobility group box chromosomal protein 1 plays a role in the pathogenesis of rheumatoid arthritis as a novel cytokine. Arthritis Rheum 48, 971-981 (2003).
36. Pullerits, R., et al. High mobility group box chromosomal protein 1, a DNA binding cytokine, induces arthritis. Arthritis Rheum 48, 1693-1700 (2003).
37. Kokkola, R., et al. Successful treatment of collagen-induced arthritis in mice and rats by targeting extracellular high mobility group box chromosomal protein 1 activity. Arthritis Rheum 48, 2052-2058 (2003).
38. Gutowski, N.J., Newcombe, J. & Cuzner, M.L. Tenascin-R and C in multiple sclerosis lesions: relevance to extracellular matrix remodelling. Neuropathol Appl Neurobiol 25, 207-214 (1999).
39. Amin, K., et al. Inflammation and structural changes in the airways of patients with primary Sjogren's syndrome. Respir Med 95, 904-910 (2001).
40. Loots, M.A., et al. Differences in cellular infiltrate and extracellular matrix of chronic diabetic and venous ulcers versus acute wounds. J Invest Dermatol 111, 850-857 (1998).
41. Lange, K., et al. Endothelin receptor type B counteracts tenascin-C-induced endothelin receptor type A-dependent focal adhesion and actin stress fiber disorganization. Cancer Res 67, 6163-6173 (2007).
42. Saga, Y., Yagi, T., Ikawa, Y., Sakakura, T. & Aizawa, S. Mice develop normally without tenascin. Genes Dev 6, 1821-1831 (1992).
43. Hoshino, K., et al. Cutting edge: Toll-like receptor 4 (TLR4)-deficient mice are hyporesponsive to lipopolysaccharide: evidence for TLR4 as the Lps gene product. J Immunol 162, 3749-3752 (1999).
44. Takeuchi, O., et al. Differential roles of TLR2 and TLR4 in recognition of gram-negative and gram-positive bacterial cell wall components. Immunity 11, 443-451 (1999).
45. Keystone, E.C., Schorlemmer, H.U., Pope, C. & Allison, A.C. Zymosan-induced arthritis: a model of chronic proliferative arthritis following activation of the alternative pathway of complement. Arthritis Rheum 20, 1396-1401 (1977).
46. van Lent, P.L., et al. Fcgamma receptors directly mediate cartilage, but not bone, destruction in murine antigen-induced arthritis: uncoupling of cartilage damage from bone erosion and joint inflammation. Arthritis Rheum 54, 3868-3877 (2006).
47. Foxwell, B., et al. Efficient adenoviral infection with IkappaB alpha reveals that macrophage tumor necrosis factor alpha production in rheumatoid arthritis is NF-kappaB dependent. Proc Natl Acad Sci U S A 95, 8211-8215 (1998).
48. Kurt-Jones, E.A., et al. Use of murine embryonic fibroblasts to define Toll-like receptor activation and specificity. J Endotoxin Res 10, 419-424 (2004).
49. Todaro, G.J. & Green, H. Quantitative studies of the growth of mouse embryo cells in culture and their development into established lines. J Cell Biol 17, 299-313 (1963).
50. Butler, D.M., Malfait, A.M., Maini, R.N., Brennan, F.M. & Feldmann, M. Anti-IL-12 and anti-TNF antibodies synergistically suppress the progression of murine collagen-induced arthritis. Eur J Immunol 29, 2205-2212 (1999).
51. Ho, S.N., Hunt, H.D., Horton, R.M., Pullen, J.K. & Pease, L.R. Site-directed mutagenesis by overlap extension using the polymerase chain reaction. Gene 77, 51-59 (1989).
52. Clark, R.A., Erickson, H.P. & Springer, T.A. Tenascin supports lymphocyte rolling. J Cell Biol 137, 755-765 (1997).
53. El-Karef, A., et al. Deficiency of tenascin-C attenuates liver fibrosis in immune-mediated chronic hepatitis in mice. J Pathol 211, 86-94 (2007).
54. Loike, J.D., Cao, L., Budhu, S., Hoffman, S. & Silverstein, S.C. Blockade of alpha 5 beta 1 integrins reverses the inhibitory effect of tenascin on chemotaxis of human monocytes and polymorphonuclear leukocytes through three-dimensional gels of extracellular matrix proteins. J Immunol 166, 7534-7542 (2001).
55. Talts, J.F., Wirl, G., Dictor, M., Muller, W.J. & Fassler, R. tenascin-C modulates tumor stroma and monocyte/macrophage recruitment but not tumor growth or metastasis in a mouse strain with spontaneous mammary cancer. J Cell Sci 112 (Pt 12), 1855-1864 (1999).
56. Jones (2000) Matrix Biol., 19, 581-96
57. Harandl (2009) Expert Review of Vaccines, 8, 293-298
58. McIntyre (2006) BMC Biotechnol. 6: 1
59. Paddison (2002) Genes Dev. 16 (8): 948-58
60. Andreakos (2004) Blood, 103, 2229-37
61. Goh, F.G., Piccinini, A.M., Krausgruber, T., Udalova, I.A. & Midwood, K.S. Transcriptional regulation of the endogenous danger signal tenascin-C: a novel autocrine loop in inflammation. J Immunol 184, 2655-2662 (2010).
62. Midwood, K. et al. Tenascin-C is an endogenous activator of Toll-like receptor 4 that is essential for maintaining inflammation in arthritic joint disease. Nat Med 15, 774-780 (2009).
63. LaFleur, D.W. et al. Aortic smooth muscle cells interact with tenascin-C through its fibrinogen-like domain. J Biol Chem 272, 32798-32803 (1997).
64. Taylor, P.C. & Feldmann, M. Anti-TNF biologic agents: still the therapy of choice for rheumatoid arthritis. Nat Rev Rheumatol 5, 578-582 (2009).
65. Yokoyama, K., Erickson, H.P., Ikeda, Y. & Takada, Y. Identification of amino acid sequences in fibrinogen gamma -chain and tenascin C C-terminal domains critical for binding to integrin alpha vbeta 3. J Biol Chem 275, 16891-16898 (2000).

## Claims

1. An agent for use in the treatment of chronic inflammations selected from rheumatoid arthritis and Sjogren's disease, wherein the agent modulates the biological activity of tenascin-C by inhibiting the binding properties of Tenascin C, and the agent is an antibody or antigen-binding fragment thereof having specificity to FBG domain that activates TLR4, wherein said antibody or antigen-binding fragment thereof specifically binds to at least one peptide selected from:
• Peptide 5 ELRVDLRDHGETAFAVYDKFSVGDAKTRYK
• Peptide 6 KTRYKLKVEGYSGTAGDSMAYHNGRSFST, and
• Peptide 7 RSFSTFDKDTDSAITNCALSYKGAFWYRN.

2. An agent for use according to claim 1, wherein the antigen-binding fragment thereof is selected from the group consisting of Fv fragments, scFv fragments, Fab, single variable domains and domain antibodies.

3. An agent for use according to anyone of claims 1 to 2, wherein the antibody or antigen binding fragment thereof is humanised.

4. A composition comprising an agent as defined in any one of claims 1 to 3, and a pharmaceutically acceptable carrier, excipient and/or diluent for use in the treatment of chronic inflammations selected from rheumatoid arthritis and Sjogren's disease.

5. A composition for use according to claim 4, wherein the composition further comprises at least one other agent

6. A composition for use according to claim 5, wherein the at least one agent is an anti-inflammatory agent

7. A composition for use according to claim 6, wherein the anti-inflammatory agent is selected from the group consisting of a non-steroidal anti-inflammatory, corticosteroids, disease-modifying antirheumatic drugs or immunosuppressants, a statin, a biological agent, an immunosuppressive agent, a salicylate and a microbicidal agent.

## Patentansprüche

1. Mittel zur Verwendung bei der Behandlung chronischer Entzündungen, die aus rheumatoider Arthritis und Sjögren-Syndrom ausgewählt sind, wobei das Mittel die biologische Aktivität von Tenascin-C durch Hemmen der Bindungseigenschaften von Tenascin C moduliert und das Mittel ein Antikörper oder ein antigenbindendes Fragment davon ist, das eine Spezifität für eine FBG-Domäne aufweist, die TLR4 aktiviert, wobei der Antikörper oder das antigenbindende Fragment davon an wenigstens ein Peptid spezifisch bindet, das aus Folgendem ausgewählt ist:
• Peptid 5 ELRVDLRDHGETAFAVYDKFSVGDAKTRYK
• Peptid 6 KTRYKLKVEGYSGTAGDSMAYHNGRSFST, und
• Peptid 7 RSFSTFDKDTDSAITNCALSYKGAFWYRN.

2. Mittel zur Verwendung nach Anspruch 1, wobei das antigenbindende Fragment davon aus der Gruppe ausgewählt ist, die aus Fv-Fragmenten, scFv-Fragmenten, Fab, einzelnen variablen Domänen und Domänen-Antikörpern besteht.

3. Mittel zur Verwendung nach einem der Ansprüche 1 bis 2, wobei der Antikörper oder das antigenbindende Fragment davon humanisiert ist.

4. Zusammensetzung, die ein Mittel nach einem der Ansprüche 1 bis 3 und eine pharmazeutisch unbedenkliche Trägersubstanz, einen Arzneistoffträger und/oder ein Verdünnungsmittel zur Verwendung bei der Behandlung von chronischen Entzündungen umfasst, die aus rheumatoider Arthritis und Sjögren-Syndrom ausgewählt sind.

5. Zusammensetzung zur Verwendung nach Anspruch 4, wobei die Zusammensetzung ferner wenigstens ein anderes Mittel umfasst.

6. Zusammensetzung zur Verwendung nach Anspruch 5, wobei das wenigstens eine Mittel ein entzündungshemmendes Mittel ist.

7. Zusammensetzung zur Verwendung nach Anspruch 6, wobei das entzündungshemmende Mittel aus der Gruppe ausgewählt ist, die aus einem nichtsteroidalen entzündungshemmenden Mittel, Kortikosteroiden, krankheitsmodifizierenden antirheumatischen Arzneimitteln oder Immunsuppressiva, einem Statin, einem biologischen Mittel, einem immunsuppressiven Mittel, einem Salicylat und einem mikrobiziden Mittel besteht.

## Revendications

1. Agent destiné à être utilisé dans le traitement d'inflammations chroniques choisies parmi la polyarthrite rhumatoïde et la maladie de Sjögren, l'agent modulant l'activité biologique de la ténascine-C en inhibant les propriétés de liaison de la ténascine C, et l'agent étant un anticorps ou un fragment de liaison à l'antigène de celle-ci ayant une spécificité pour le domaine FBG qui active TLR4, ledit anticorps ou fragment de liaison à l'antigène de celui-ci se liant spécifiquement à au moins un peptide choisi parmi :
• Peptide 5 ELRVDLRDHGETAFAVYDKFSVGDAKTRYK
• Peptide 6 KTRYKLKVEGYSGTAGDSMAYHNGRSFST, et
• Peptide 7 RSFSTFDKDTDSAITNCALSYKGAFWYRN.

2. Agent destiné à être utilisé selon la revendication 1, son fragment de liaison à l'antigène étant choisi dans le groupe constitué de fragments Fv, fragments scFv, Fab, de domaines variables uniques et d'anticorps de domaine.

3. Agent destiné à être utilisé selon l'une quelconque des revendications 1 à 2, l'anticorps ou le fragment de liaison à l'antigène de celui-ci étant humanisé.

4. Composition comprenant un agent selon l'une quelconque des revendications 1 à 3, et un support, excipient et/ou diluant pharmaceutiquement acceptable destiné à être utilisé dans le traitement d'inflammations chroniques choisies parmi la polyarthrite rhumatoïde et la maladie de Sjögren.

5. Composition destinée à être utilisée selon la revendication 4, la composition comprenant en outre au moins un autre agent.

6. Composition destinée à être utilisée selon la revendication 5, l'au moins un agent étant un agent anti-inflammatoire.

7. Composition destinée à être utilisée selon la revendication 6, l'agent anti-inflammatoire étant choisi dans le groupe constitué d'un agent anti-inflammatoire non stéroïdien, des corticostéroïdes, des médicaments antirhumatismaux ou immunosuppresseurs, une statine, un agent biologique, un agent immunosuppresseur, un salicylate et un agent microbicide.


### SEQUENCE LISTING

<110> Imperial Innovations Limited
<120> Biological materials and uses thereof
<130> ICOBH/P45612PC
<140> PCT/GB2010/000460
   <141> 15th March 2010
<150> GB0904355.5
   <151> 13 March 2009
<160> 37
<170> SeqWin99
<210> 1
   <211> 51
   <212> DNA
   <213> Artificial Sequence
<220>
   <223> TA Forward primer
<400> 1
   atacatatgc atcatcatca tcatcatggg gtcctcaaga aagtcatccg g 51
<210> 2
   <211> 33
   <212> DNA
   <213> Artificial Sequence
<220>
   <223> TA Reverse primer
<400> 2
   gccggatcct tagcctgctc ctgcagtaca ttg 33
<210> 3
   <211> 47
   <212> DNA
   <213> Artificial Sequence
<220>
   <223> EGF-L PCR1 Forward primer
<400> 3
   acagtggtac caccatgggg gccatggggg ccatgactca gctgttg 47
<210> 4
   <211> 45
   <212> DNA
   <213> Artificial Sequence
<220>
   <223> EGF-L PCR1 Reverse primer
<400> 4
   cttgtcatcg tcgtccttgt agtcaccttc ggtagcgagg gcaag 45
<210> 5
   <211> 44
   <212> DNA
   <213> Artificial Sequence
<220>
   <223> EGF-L PCR2 Forward primer
<400> 5
   gactagaagg acgacgatga caagtgctgt ctccagcctg ccac 44
<210> 6
   <211> 56
   <212> DNA
   <213> Artificial Sequence
<220>
   <223> EGF-L PCR2 Reverse primer
<400> 6
   gacagcggat ccttaatgat gatgatgatg atgtgagcag tcttctccgc tgtagc 56
<210> 7
   <211> 47
   <212> DNA
   <213> Artificial Sequence
<220>
   <223> TN1-5 Forward primer
<400> 7
   atacatatgc atcatcatca tcatcatgag gtgtctcctc ccaaaga 47
<210> 8
   <211> 33
   <212> DNA
   <213> Artificial Sequence
<220>
   <223> TN1-5 Reverse primer
<400> 8
   gccggtacct taagtggatg ccttcacacg tgc 33
<210> 9
   <211> 47
   <212> DNA
   <213> Artificial Sequence
<220>
   <223> TN1-3 Forward primer
<400> 9
   atacatatgc atcatcatca tcatcatgag gtgtctcctc ccaaaga 47
<210> 10
   <211> 33
   <212> DNA
   <213> Artificial Sequence
<220>
   <223> TN1-3 Reverse primer
<400> 10
   gccggtacct tatgttgtga aggtctcttt ggc 33
<210> 11
   <211> 50
   <212> DNA
   <213> Artificial Sequence
<220>
   <223> TN3-5 Forward primer
<400> 11
   atacatatgc atcatcatca tcatcatcgc ttggatgccc ccagccagat 50
<210> 12
   <211> 33
   <212> DNA
   <213> Artificial Sequence
<220>
   <223> TN3-5 Reverse primer
<400> 12
   gccggtacct taagtggatg ccttcacacg tgc 33
<210> 13
   <211> 48
   <212> DNA
   <213> Artificial Sequence
<220>
   <223> TN5-7 Forward primer
<400> 13
   atacatatgc atcatcatca tcatcatgag ttggacacgc ccaaggac 48
<210> 14
   <211> 38
   <212> DNA
   <213> Artificial Sequence
<220>
   <223> TN5-7 Reverse primer
<400> 14
   gccggatcct tatgttgtga acttggcagt gatggttg 38
<210> 15
   <211> 48
   <212> DNA
   <213> Artificial Sequence
<220>
   <223> TN6-8 Forward primer
<400> 15
   atacatatgc atcatcatca tcatcatgcc atgggctccc caaaggaa 48
<210> 16
   <211> 36
   <212> DNA
   <213> Artificial Sequence
<220>
   <223> TN6-8 Reverse primer
<400> 16
   gccggatcct tatgtggtga agatggtctg gatcat 36
<210> 17
   <211> 49
   <212> DNA
   <213> Artificial Sequence
<220>
   <223> FBG Forward primer
<400> 17
   atacatatgc atcatcatca tcatcatatt ggactcctgt accccttcc 49
<210> 18
   <211> 35
   <212> DNA
   <213> Artificial Sequence
<220>
   <223> FBG Reverse primer
<400> 18
   gccggatcct tatgcccgtt tgcgcctgcc ttcaa 35
<210> 19
   <211> 18
   <212> DNA
   <213> Artificial Sequence
<220>
   <223> His tag
<400> 19
   catcatcatc atcatcat 18
<210> 20
   <211> 30
   <212> PRT
   <213> Homo sapiens
<400> 20
   Thr Ile Gly Leu Leu Tyr Pro Phe Pro Lys Asp Cys Ser Gln Ala Met
   1               5                   10                  15
   Leu Asn Gly Asp Thr Thr Ser Gly Leu Tyr Thr Ile Tyr Leu
               20                  25                  30
<210> 21
   <211> 30
   <212> PRT
   <213> Homo sapiens
<400> 21
   Tyr Thr Ile Tyr Leu Asn Gly Asp Lys Ala Glu Ala Leu Glu Val Phe
   1               5                   10                  15
   Cys Asp Met Thr Ser Asp Gly Gly Gly Trp Ile Val Phe Leu
               20                  25                  30
<210> 22
   <211> 30
   <212> PRT
   <213> Homo sapiens
<400> 22
   Trp Ile Val Phe Leu Arg Arg Lys Asn Gly Arg Glu Asn Phe Tyr Gln
   1               5                   10                  15
   Asn Trp Lys Ala Tyr Ala Ala Gly Phe Gly Asp Arg Arg Glu
               20                  25                  30
<210> 23
   <211> 28
   <212> PRT
   <213> Homo sapiens
<400> 23
   Gly Asp Arg Arg Glu Glu Phe Trp Leu Gly Leu Asp Asn Leu Asn Lys
   1               5                   10                  15
   Ile Thr Ala Gln Gly Gln Tyr Glu Leu Arg Val Asp
               20                  25
<210> 24
   <211> 30
   <212> PRT
   <213> Homo sapiens
<400> 24
   Glu Leu Arg Val Asp Leu Arg Asp His Gly Glu Thr Ala Phe Ala Val
   1               5                   10                  15
   Tyr Asp Lys Phe Ser Val Gly Asp Ala Lys Thr Arg Tyr Lys
               20                  25                  30
<210> 25
   <211> 29
   <212> PRT
   <213> Artificial Sequence
<220>
   <223> Homo sapiens
<400> 25
   Lys Thr Arg Tyr Lys Leu Lys Val Glu Gly Tyr Ser Gly Thr Ala Gly
   1               5                   10                  15
   Asp Ser Met Ala Tyr His Asn Gly Arg Ser Phe Ser Thr
               20                  25
<210> 26
   <211> 29
   <212> PRT
   <213> Artificial Sequence
<220>
   <223> Homo sapiens
<400> 26
   Arg Ser Phe Ser Thr Phe Asp Lys Asp Thr Asp Ser Ala Ile Thr Asn
   1               5                   10                  15
   Cys Ala Leu Ser Tyr Lys Gly Ala Phe Trp Tyr Arg Asn
   20                  25
<210> 27
   <211> 30
   <212> PRT
   <213> Artificial Sequence
<220>
   <223> Homo sapiens
<400> 27
   Trp Tyr Arg Asn Cys His Arg Val Asn Leu Met Gly Arg Tyr Gly Asp
   1               5                   10                  15
   Asn Asn His Ser Gln Gly Val Asn Trp Phe His Trp Lys Gly
               20                  25                  30
<210> 28
   <211> 32
   <212> PRT
   <213> Artificial Sequence
<220>
   <223> Homo sapiens
<400> 28
   Phe His Trp Lys Gly His Glu His Ser Ile Gln Phe Ala Glu Met Lys
   1               5                   10                  15
   Leu Arg Pro Ser Asn Phe Arg Asn Leu Glu Gly Arg Arg Lys Arg Ala
               20                  25                  30
<210> 29
   <211> 21
   <212> DNA
   <213> Artificial Sequence
<220>
   <223> s7069 siRNA sense strand
<220>
   <221> misc_feature
   <223> /note="Combined DNA / RNA"
<400> 29
   cgcgagaacu ucuaccaaat t 21
<210> 30
   <211> 21
   <212> DNA
   <213> Artificial Sequence
<220>
   <223> s7069 siRNA antisense strand
<220>
   <221> misc_feature
   <223> /note="Combined DNA / RNA"
<400> 30
   uuugguagaa guucucgcgt c 21
<210> 31
   <211> 21
   <212> DNA
   <213> Artificial Sequence
<220>
   <223> ss229491 siRNA sense strand
<220>
   <221> misc_feature
   <223> /note="Combined DNA / RNA"
<400> 31
   ggaauaugaa uaaagaagat t 21
<210> 32
   <211> 21
   <212> DNA
   <213> Artificial Sequence
<220>
   <223> s229491 siRNA antisense strand
<220>
   <221> misc_feature
   <223> /note="Combined DNA / RNA"
<400> 32
   ucuucuuuau ucauauuccg g 21
<210> 33
   <211> 23
   <212> PRT
   <213> Homo sapiens
<400> 33
   Gly Val Leu Lys Lys Val Ile Arg His Lys Arg Gln Ser Gly Val Asn
   1               5                   10                  15
   Ala Thr Leu Pro Glu Glu Asn
               20
<210> 34
   <211> 476
   <212> PRT
   <213> Homo sapiens
<400> 34
   Cys Cys Leu Gln Pro Ala Thr Gly Arg Leu Asp Thr Arg Pro Phe Cys
   1               5                   10                  15
   Ser Gly Arg Gly Asn Phe Ser Thr Glu Gly Cys Gly Cys Val Cys Glu
               20                  25                  30
   Pro Gly Trp Lys Gly Pro Asn Cys Ser Glu Pro Glu Cys Pro Gly Asn
           35                  40                  45
   Cys His Leu Arg Gly Arg Cys Ile Asp Gly Gln Cys Ile Cys Asp Asp
       50                  55                  60
   Gly Phe Thr Gly Glu Asp Cys Ser Gln Leu Ala Cys Pro Ser Asp Cys
   65                  70                  75                  80
   Asn Asp Gln Gly Lys Cys Val Asn Gly Val Cys Ile Cys Phe Glu Gly
                   85                  90                  95
   Tyr Ala Gly Ala Asp Cys Ser Arg Glu Ile Cys Pro Val Pro Cys Ser
               100                 105                 110
   Glu Glu His Gly Thr Cys Val Asp Gly Leu Cys Val Cys His Asp Gly
           115                 120                 125
   Phe Ala Gly Asp Asp Cys Asn Lys Pro Leu Cys Leu Asn Asn Cys Tyr
       130                 135                 140
   Asn Arg Gly Arg Cys Val Glu Asn Glu Cys Val Cys Asp Glu Gly Phe
   145                 150                 155                 160
   Thr Gly Glu Asp Cys Ser Glu Leu Ile Cys Pro Asn Asp Cys Phe Asp
                   165                 170                 175
   Arg Gly Arg Cys Ile Asn Gly Thr Cys Tyr Cys Glu Glu Gly Phe Thr
               180                 185                 190
   Gly Glu Asp Cys Gly Lys Pro Thr Cys Pro His Ala Cys His Thr Gln
           195                 200                 205
   Gly Arg Cys Glu Glu Gly Gln Cys Val Cys Asp Glu Gly Phe Ala Gly
       210                 215                 220
   Leu Asp Cys Ser Glu Lys Arg Cys Pro Ala Asp Cys His Asn Arg Gly
   225                 230                 235                 240
   Arg Cys Val Asp Gly Arg Cys Glu Cys Asp Asp Gly Phe Thr Gly Ala
                   245                 250                 255
   Asp Cys Gly Glu Leu Lys Cys Pro Asn Gly Cys Ser Gly His Gly Arg
               260                 265                 270
   Cys Val Asn Gly Gln Cys Val Cys Asp Glu Gly Tyr Thr Gly Glu Asp
           275                 280                 285
   Cys Ser Gln Leu Arg Cys Pro Asn Asp Cys His Ser Arg Gly Arg Cys
       290                 295                 300
   Val Glu Gly Lys Cys Val Cys Glu Gln Gly Phe Lys Gly Tyr Asp Cys
   305                 310                 315                 320
   Ser Asp Met Ser Cys Pro Asn Asp Cys His Gln His Gly Arg Cys Val
                   325                 330                 335
   Asn Gly Met Cys Val Cys Asp Asp Gly Tyr Thr Gly Glu Asp Cys Arg
               340                 345                 350
   Asp Arg Gln Cys Pro Arg Asp Cys Ser Asn Arg Gly Leu Cys Val Asp
           355                 360                 365
   Gly Gln Cys Val Cys Glu Asp Gly Phe Thr Gly Pro Asp Cys Ala Glu
       370                 375                 380
   Leu Ser Cys Pro Asn Asp Cys His Gly Gln Gly Arg Cys Val Asn Gly
   385                 390                 395                 400
   Gln Cys Val Cys His Glu Gly Phe Met Gly Lys Asp Cys Lys Glu Gln
                   405                 410                 415
   Arg Cys Pro Ser Asp Cys His Gly Gln Gly Arg Cys Val Asp Gly Gln
               420                 425                 430
   Cys Ile Cys His Glu Gly Phe Thr Gly Leu Asp Cys Gly Gln His Ser
           435                 440                 445
   Cys Pro Ser Asp Cys Asn Asn Leu Gly Gln Cys Val Ser Gly Arg Cys
       450                 455                 460
   Ile Cys Asn Glu Gly Tyr Ser Gly Glu Asp Cys Ser
   465                 470                 475
<210> 35
   <211> 1352
   <212> PRT
   <213> Homo sapiens
<400> 35
   Glu Val Ser Pro Pro Lys Asp Leu Val Val Thr Glu Val Thr Glu Glu
   1               5                   10                  15
   Thr Val Asn Leu Ala Trp Asp Asn Glu Met Arg Val Thr Glu Tyr Leu
               20                  25                  30
   Val Val Tyr Thr Pro Thr His Glu Gly Gly Leu Glu Met Gln Phe Arg
           35                  40                  45
   Val Pro Gly Asp Gln Thr Ser Thr Ile Ile Gln Glu Leu Glu Pro Gly
       50                  55                  60
   Val Glu Tyr Phe Ile Arg Val Phe Ala Ile Leu Glu Asn Lys Lys Ser
   65                  70                  75                  80
   Ile Pro Val Ser Ala Arg Val Ala Thr Tyr Leu Pro Ala Pro Glu Gly
                   85                  90                  95
   Leu Lys Phe Lys Ser Ile Lys Glu Thr Ser Val Glu Val Glu Trp Asp
               100                 105                 110
   Pro Leu Asp Ile Ala Phe Glu Thr Trp Glu Ile Ile Phe Arg Asn Met
           115                 120                 125
   Asn Lys Glu Asp Glu Gly Glu Ile Thr Lys Ser Leu Arg Arg Pro Glu
       130                 135                 140
   Thr Ser Tyr Arg Gln Thr Gly Leu Ala Pro Gly Gln Glu Tyr Glu Ile
   145                 150                 155                 160
   Ser Leu His Ile Val Lys Asn Asn Thr Arg Gly Pro Gly Leu Lys Arg
                   165                 170                 175
   Val Thr Thr Thr Arg Leu Asp Ala Pro Ser Gln Ile Glu Val Lys Asp
               180                 185                 190
   Val Thr Asp Thr Thr Ala Leu Ile Thr Trp Phe Lys Pro Leu Ala Glu
           195                 200                 205
   Ile Asp Gly Ile Glu Leu Thr Tyr Gly Ile Lys Asp Val Pro Gly Asp
       210                 215                 220
   Arg Thr Thr Ile Asp Leu Thr Glu Asp Glu Asn Gln Tyr Ser Ile Gly
   225                 230                 235                 240
   Asn Leu Lys Pro Asp Thr Glu Tyr Glu Val Ser Leu Ile Ser Arg Arg
                   245                 250                 255
   Gly Asp Met Ser Ser Asn Pro Ala Lys Glu Thr Phe Thr Thr Gly Leu
               260                 265                 270
   Asp Ala Pro Arg Asn Leu Arg Arg Val Ser Gln Thr Asp Asn Ser Ile
           275                 280                 285
   Thr Leu Glu Trp Arg Asn Gly Lys Ala Ala Ile Asp Ser Tyr Arg Ile
       290                 295                 300
   Lys Tyr Ala Pro Ile Ser Gly Gly Asp His Ala Glu Val Asp Val Pro
   305                 310                 315                 320
   Lys Ser Gln Gln Ala Thr Thr Lys Thr Thr Leu Thr Gly Leu Arg Pro
                   325                 330                 335
   Gly Thr Glu Tyr Gly Ile Gly Val Ser Ala Val Lys Glu Asp Lys Glu
               340                 345                 350
   Ser Asn Pro Ala Thr Ile Asn Ala Ala Thr Glu Leu Asp Thr Pro Lys
           355                 360                 365
   Asp Leu Gln Val Ser Glu Thr Ala Glu Thr Ser Leu Thr Leu Leu Trp
       370                 375                 380
   Lys Thr Pro Leu Ala Lys Phe Asp Arg Tyr Arg Leu Asn Tyr Ser Leu
   385                 390                 395                 400
   Pro Thr Gly Gln Trp Val Gly Val Gln Leu Pro Arg Asn Thr Thr Ser
                   405                 410                 415
   Tyr Val Leu Arg Gly Leu Glu Pro Gly Gln Glu Tyr Asn Val Leu Leu
               420                 425                 430
   Thr Ala Glu Lys Gly Arg His Lys Ser Lys Pro Ala Arg Val Lys Ala
           435                 440                 445
   Ser Thr Glu Gln Ala Pro Glu Leu Glu Asn Leu Thr Val Thr Glu Val
       450                 455                 460
   Gly Trp Asp Gly Leu Arg Leu Asn Trp Thr Ala Ala Asp Gln Ala Tyr
   465                 470                 475                 480
   Glu His Phe Ile Ile Gln Val Gln Glu Ala Asn Lys Val Glu Ala Ala
                   485                 490                 495
   Arg Asn Leu Thr Val Pro Gly Ser Leu Arg Ala Val Asp Ile Pro Gly
               500                 505                 510
   Leu Lys Ala Ala Thr Pro Tyr Thr Val Ser Ile Tyr Gly Val Ile Gln
           515                 520                 525
   Gly Tyr Arg Thr Pro Val Leu Ser Ala Glu Ala Ser Thr Gly Glu Thr
       530                 535                 540
   Pro Asn Leu Gly Glu Val Val Val Ala Glu Val Gly Trp Asp Ala Leu
   545                 550                 555                 560
   Lys Leu Asn Trp Thr Ala Pro Glu Gly Ala Tyr Glu Tyr Phe Phe Ile
                   565                 570                 575
   Gln Val Gln Glu Ala Asp Thr Val Glu Ala Ala Gln Asn Leu Thr Val
               580                 585                 590
   Pro Gly Gly Leu Arg Ser Thr Asp Leu Pro Gly Leu Lys Ala Ala Thr
           595                 600                 605
   His Tyr Thr Ile Thr Ile Arg Gly Val Thr Gln Asp Phe Ser Thr Thr
       610                 615                 620
   Pro Leu Ser Val Glu Val Leu Thr Glu Glu Val Pro Asp Met Gly Asn
   625                 630                 635                 640
   Leu Thr Val Thr Glu Val Ser Trp Asp Ala Leu Arg Leu Asn Trp Thr
                   645                 650                 655
   Thr Pro Asp Gly Thr Tyr Asp Gln Phe Thr Ile Gln Val Gln Glu Ala
               660                 665                 670
   Asp Gln Val Glu Glu Ala His Asn Leu Thr Val Pro Gly Ser Leu Arg
           675                 680                 685
   Ser Met Glu Ile Pro Gly Leu Arg Ala Gly Thr Pro Tyr Thr Val Thr
       690                 695                 700
   Leu His Gly Glu Val Arg Gly His Ser Thr Arg Pro Leu Ala Val Glu
   705                 710                 715                 720
   Val Val Thr Glu Asp Leu Pro Gln Leu Gly Asp Leu Ala Val Ser Glu
                   725                 730                 735
   Val Gly Trp Asp Gly Leu Arg Leu Asn Trp Thr Ala Ala Asp Asn Ala
               740                 745                 750
   Tyr Glu His Phe Val Ile Gln Val Gln Glu Val Asn Lys Val Glu Ala
           755                 760                 765
   Ala Gln Asn Leu Thr Leu Pro Gly Ser Leu Arg Ala Val Asp Ile Pro
       770                 775                 780
   Gly Leu Glu Ala Ala Thr Pro Tyr Arg Val Ser Ile Tyr Gly Val Ile
   785                 790                 795                 800
   Arg Gly Tyr Arg Thr Pro Val Leu Ser Ala Glu Ala Ser Thr Ala Lys
                   805                 810                 815
   Glu Pro Glu Ile Gly Asn Leu Asn Val Ser Asp Ile Thr Pro Glu Ser
               820                 825                 830
   Phe Asn Leu Ser Trp Met Ala Thr Asp Gly Ile Phe Glu Thr Phe Thr
           835                 840                 845
   Ile Glu Ile Ile Asp Ser Asn Arg Leu Leu Glu Thr Val Glu Tyr Asn
       850                 855                 860
   Ile Ser Gly Ala Glu Arg Thr Ala His Ile Ser Gly Leu Pro Pro Ser
   865                 870                 875                 880
   Thr Asp Phe Ile Val Tyr Leu Ser Gly Leu Ala Pro Ser Ile Arg Thr
                   885                 890                 895
   Lys Thr Ile Ser Ala Thr Ala Thr Thr Glu Ala Leu Pro Leu Leu Glu
               900                 905                 910
   Asn Leu Thr Ile Ser Asp Ile Asn Pro Tyr Gly Phe Thr Val Ser Trp
   915 920 925
   Met Ala Ser Glu Asn Ala Phe Asp Ser Phe Leu Val Thr Val Val Asp
   930 935 940
   Ser Gly Lys Leu Leu Asp Pro Gln Glu Phe Thr Leu Ser Gly Thr Gln
   945 950 955 960
   Arg Lys Leu Glu Leu Arg Gly Leu Ile Thr Gly Ile Gly Tyr Glu Val
   965 970 975
   Met Val Ser Gly Phe Thr Gln Gly His Gln Thr Lys Pro Leu Arg Ala
   980 985 990
   Glu Ile Val Thr Glu Ala Glu Pro Glu Val Asp Asn Leu Leu Val Ser
   995 1000 1005
   Asp Ala Thr Pro Asp Gly Phe Arg Leu Ser Trp Thr Ala Asp Glu Gly
   1010 1015 1020
   Val Phe Asp Asn Phe Val Leu Lys Ile Arg Asp Thr Lys Lys Gln Ser
   1025 1030 1035 1040
   Glu Pro Leu Glu Ile Thr Leu Leu Ala Pro Glu Arg Thr Arg Asp Leu
   1045 1050 1055
   Thr Gly Leu Arg Glu Ala Thr Glu Tyr Glu Ile Glu Leu Tyr Gly Ile
   1060 1065 1070
   Ser Lys Gly Arg Arg Ser Gln Thr Val Ser Ala Ile Ala Thr Thr Ala
   1075 1080 1085
   Met Gly Ser Pro Lys Glu Val Ile Phe Ser Asp Ile Thr Glu Asn Ser
   1090 1095 1100
   Ala Thr Val Ser Trp Arg Ala Pro Thr Ala Gln Val Glu Ser Phe Arg
   1105 1110 1115 1120
   Ile Thr Tyr Val Pro Ile Thr Gly Gly Thr Pro Ser Met Val Thr Val
   1125 1130 1135
   Asp Gly Thr Lys Thr Gln Thr Arg Leu Val Lys Leu Ile Pro Gly Val
   1140 1145 1150
   Glu Tyr Leu Val Ser Ile Ile Ala Met Lys Gly Phe Glu Glu Ser Glu
   1155 1160 1165
   Pro Val Ser Gly Ser Phe Thr Thr Ala Leu Asp Gly Pro Ser Gly Leu
   1170 1175 1180
   Val Thr Ala Asn Ile Thr Asp Ser Glu Ala Leu Ala Arg Trp Gln Pro
   1185 1190 1195 1200
   Ala Ile Ala Thr Val Asp Ser Tyr Val Ile Ser Tyr Thr Gly Glu Lys
   1205 1210 1215
   Val Pro Glu Ile Thr Arg Thr Val Ser Gly Asn Thr Val Glu Tyr Ala
   1220 1225 1230
   Leu Thr Asp Leu Glu Pro Ala Thr Glu Tyr Thr Leu Arg Ile Phe Ala
   1235 1240 1245
   Glu Lys Gly Pro Gln Lys Ser Ser Thr Ile Thr Ala Lys Phe Thr Thr
   1250 1255 1260
   Asp Leu Asp Ser Pro Arg Asp Leu Thr Ala Thr Glu Val Gln Ser Glu
   1265 1270 1275 1280
   Thr Ala Leu Leu Thr Trp Arg Pro Pro Arg Ala Ser Val Thr Gly Tyr
   1285 1290 1295
   Leu Leu Val Tyr Glu Ser Val Asp Gly Thr Val Lys Glu Val Ile Val
   1300 1305 1310
   Gly Pro Asp Thr Thr Ser Tyr Ser Leu Ala Asp Leu Ser Pro Ser Thr
   1315 1320 1325
   His Tyr Thr Ala Lys Ile Gln Ala Leu Asn Gly Pro Leu Arg Ser Asn
   1330 1335 1340
   Met Ile Gln Thr Ile Phe Thr Thr
   1345 1350
<210> 36
   <211> 228
   <212> PRT
   <213> Homo sapiens
<400> 36
   Ile Gly Leu Leu Tyr Pro Phe Pro Lys Asp Cys Ser Gln Ala Met Leu
   1               5                   10                  15
   Asn Gly Asp Thr Thr Ser Gly Leu Tyr Thr Ile Tyr Leu Asn Gly Asp
               20                  25                  30
   Lys Ala Gln Ala Leu Glu Val Phe Cys Asp Met Thr Ser Asp Gly Gly
           35                  40                  45
   Gly Trp Ile Val Phe Leu Arg Arg Lys Asn Gly Arg Glu Asn Phe Tyr
       50                  55                  60
   Gln Asn Trp Lys Ala Tyr Ala Ala Gly Phe Gly Asp Arg Arg Glu Glu
   65                  70                  75                  80
   Phe Trp Leu Gly Leu Asp Asn Leu Asn Lys Ile Thr Ala Gln Gly Gln
                   85                  90                  95
   Tyr Glu Leu Arg Val Asp Leu Arg Asp His Gly Glu Thr Ala Phe Ala
               100                 105                 110
   Val Tyr Asp Lys Phe Ser Val Gly Asp Ala Lys Thr Arg Tyr Lys Leu
           115                 120                 125
   Lys Val Glu Gly Tyr Ser Gly Thr Ala Gly Asp Ser Met Ala Tyr His
       130                 135                 140
   Asn Gly Arg Ser Phe Ser Thr Phe Asp Lys Asp Thr Asp Ser Ala Ile
   145                 150                 155                 160
   Thr Asn Cys Ala Leu Ser Tyr Lys Gly Ala Phe Trp Tyr Arg Asn Cys
                   165                 170                 175
   His Arg Val Asn Leu Met Gly Arg Tyr Gly Asp Asn Asn His Ser Gln
               180                 185                 190
   Gly Val Asn Trp Phe His Trp Lys Gly His Glu His Ser Ile Gln Phe
           195                 200                 205
   Ala Glu Met Lys Leu Arg Pro Ser Asn Phe Arg Asn Leu Glu Gly Arg
       210                 215                 220
   Arg Lys Arg Ala
   225
<210> 37
   <211> 7616
   <212> DNA
   <213> Homo sapiens
<400> 37
   attacagagg aaggagctcg ctatataagc cagccaaagt tggctgcacc ggccacagcc   60
   tgcctactgt cacccgcctc tcccgcgcgc agatacacgc ccccgcctcc gtgggcacaa   120
   aggcagcgct gctggggaac tcgggggaac gcgcacgtgg gaaccgccgc agctccacac   180
   tccaggtact tcttccaagg acctaggtct ctcgcccatc ggaaagaaaa taattctttc   240
   aagaagatca gggacaactg atttgaagtc tactctgtgc ttctaaatcc ccaattctgc   300
   tgaaagtgag ataccctaga gccctagagc cccagcagca cccagccaaa cccacctcca   360
   ccatgggggc catgactcag ctgttggcag gtgtctttct tgctttcctt gccctcgcta   420
   ccgaaggtgg ggtcctcaag aaagtcatcc ggcacaagcg acagagtggg gtgaacgcca   480
   ccctgccaga agagaaccag ccagtggtgt ttaaccacgt ttacaacatc aagctgccag   540
   tgggatccca gtgttcggtg gatctggagt cagccagtgg ggagaaagac ctggcaccgc   600
   cttcagagcc cagcgaaagc tttcaggagc acacagtgga tggggaaaac cagattgtct   660
   tcacacatcg catcaacatc ccccgccggg cctgtggctg tgccgcagcc cctgatgtta   720
   aggagctgct gagcagactg gaggagctgg agaacctggt gtcttccctg agggagcaat   780
   gtactgcagg agcaggctgc tgtctccagc ctgccacagg ccgcttggac accaggccct   840
   tctgtagcgg tcggggcaac ttcagcactg aaggatgtgg ctgtgtctgc gaacctggct   900
   ggaaaggccc caactgctct gagcccgaat gtccaggcaa ctgtcacctt cgaggccggt   960
   gcattgatgg gcagtgcatc tgtgacgacg gcttcacggg cgaggactgc agccagctgg   1020
   cttgccccag cgactgcaat gaccagggca agtgcgtaaa tggagtctgc atctgtttcg   1080
   aaggctacgc cggggctgac tgcagccgtg aaatctgccc agtgccctgc agtgaggagc   1140
   acggcacatg tgtagatggc ttgtgtgtgt gccacgatgg ctttgcaggc gatgactgca   1200
   acaagcctct gtgtctcaac aattgctaca accgtggacg atgcgtggag aatgagtgcg   1260
   tgtgtgatga gggtttcacg ggcgaagact gcagtgagct catctgcccc aatgactgct   1320
   tcgaccgggg ccgctgcatc aatggcacct gctactgcga agaaggcttc acaggtgaag   1380
   actgcgggaa acccacctgc ccacatgcct gccacaccca gggccggtgt gaggaggggc   1440
   agtgtgtatg tgatgagggc tttgccggtg tggactgcag cgagaagagg tgtcctgctg   1500
   actgtcacaa tcgtggccgc tgtgtagacg ggcggtgtga gtgtgatgat ggtttcactg   1560
   gagctgactg tggggagctc aagtgtccca atggctgcag tggccatggc cgctgtgtca   1620
   atgggcagtg tgtgtgtgat gagggctata ctggggagga ctgcagccag ctacggtgcc   1680
   ccaatgactg tcacagtcgg ggccgctgtg tcgagggcaa atgtgtatgt gagcaaggct   1740
   tcaagggcta tgactgcagt gacatgagct gccctaatga ctgtcaccag cacggccgct   1800
   gtgtgaatgg catgtgtgtt tgtgatgacg gctacacagg ggaagactgc cgggatcgcc   1860
   aatgccccag ggactgcagc aacaggggcc tctgtgtgga cggacagtgc gtctgtgagg   1920
   acggcttcac cggccctgac tgtgcagaac tctcctgtcc aaatgactgc catggccagg   1980
   gtcgctgtgt gaatgggcag tgcgtgtgcc atgaaggatt tatgggcaaa gactgcaagg   2040
   agcaaagatg tcccagtgac tgtcatggcc agggccgctg cgtggacggc cagtgcatct   2100
   gccacgaggg cttcacaggc ctggactgtg gccagcactc ctgccccagt gactgcaaca   2160
   acttaggaca atgcgtctcg ggccgctgca tctgcaacga gggctacagc ggagaagact   2220
   gctcagaggt gtctcctccc aaagacctcg ttgtgacaga agtgacggaa gagacggtca   2280
   acctggcctg ggacaatgag atgcgggtca cagagtacct tgtcgtgtac acgcccaccc   2340
   acgagggtgg tctggaaatg cagttccgtg tgcctgggga ccagacgtcc accatcatcc   2400
   aggagctgga gcctggtgtg gagtacttta tccgtgtatt tgccatcctg gagaacaaga   2460
   agagcattcc tgtcagcgcc agggtggcca cgtacttacc tgcacctgaa ggcctgaaat   2520
   tcaagtccat caaggagaca tctgtggaag tggagtggga tcctctagac attgcttttg   2580
   aaacctggga gatcatcttc cggaatatga ataaagaaga tgagggagag atcaccaaaa   2640
   gcctgaggag gccagagacc tcttaccggc aaactggtct agctcctggg caagagtatg   2700
   agatatctct gcacatagtg aaaaacaata cccggggccc tggcctgaag agggtgacca   2760
   ccacacgctt ggatgccccc agccagatcg aggtgaaaga tgtcacagac accactgcct   2820
   tgatcacctg gttcaagccc ctggctgaga tcgatggcat tgagctgacc tacggcatca   2880
   aagacgtgcc aggagaccgt accaccatcg atctcacaga ggacgagaac cagtactcca 2940
   tcgggaacct gaagcctgac actgagtacg aggtgtccct catctcccgc agaggtgaca 3000
   tgtcaagcaa cccagccaaa gagaccttca caacaggcct cgatgctccc aggaatcttc 3060
   gacgtgtttc ccagacagat aacagcatca ccctggaatg gaggaatggc aaggcagcta 3120
   ttgacagtta cagaattaag tatgccccca tctctggagg ggaccacgct gaggttgatg 3180
   ttccaaagag ccaacaagcc acaaccaaaa ccacactcac aggtctgagg ccgggaactg 3240
   aatatgggat tggagtttct gctgtgaagg aagacaagga gagcaatcca gcgaccatca 3300
   acgcagccac agagttggac acgcccaagg accttcaggt ttctgaaact gcagagacca 3360
   gcctgaccct gctctggaag acaccgttgg ccaaatttga ccgctaccgc ctcaattaca 3420
   gtctccccac aggccagtgg gtgggagtgc agcttccaag aaacaccact tcctatgtcc 3480
   tgagaggcct ggaaccagga caggagtaca atgtcctcct gacagccgag aaaggcagac 3540
   acaagagcaa gcccgcacgt gtgaaggcat ccactgaaca agcccctgag ctggaaaacc 3600
   tcaccgtgac tgaggttggc tgggatggcc tcagactcaa ctggaccgca gctgaccagg 3660
   cctatgagca ctttatcatt caggtgcagg aggccaacaa ggtggaggca gctcggaacc 3720
   tcaccgtgcc tggcagcctt cgggctgtgg acataccggg cctcaaggct gctacgcctt 3780
   atacagtctc catctatggg gtgatccagg gctatagaac accagtgctc tctgctgagg 3840
   cctccacagg ggaaactccc aatttgggag aggtcgtggt ggccgaggtg ggctgggatg 3900
   ccctcaaact caactggact gctccagaag gggcctatga gtactttttc attcaggtgc 3960
   aggaggctga cacagtagag gcagcccaga acctcaccgt cccaggagga ctgaggtcca 4020
   cagacctgcc tgggctcaaa gcagccactc attataccat caccatccgc ggggtcactc 4080
   aggacttcag cacaacccct ctctctgttg aagtcttgac agaggaggtt ccagatatgg 4140
   gaaacctcac agtgaccgag gttagctggg atgctctcag actgaactgg accacgccag 4200
   atggaaccta tgaccagttt actattcagg tccaggaggc tgaccaggtg gaagaggctc 4260
   acaatctcac ggttcctggc agcctgcgtt ccatggaaat cccaggcctc agggctggca 4320
   ctccttacac agtcaccctg cacggcgagg tcaggggcca cagcactcga ccccttgctg 4380
   tagaggtcgt cacagaggat ctcccacagc tgggagattt agccgtgtct gaggttggct 4440
   gggatggcct cagactcaac tggaccgcag ctgacaatgc ctatgagcac tttgtcattc 4500
   aggtgcagga ggtcaacaaa gtggaggcag cccagaacct cacgttgcct ggcagcctca 4560
   gggctgtgga catcccgggc ctcgaggctg ccacgcctta tagagtctcc atctatgggg 4620
   tgatccgggg ctatagaaca ccagtactct ctgctgaggc ctccacagcc aaagaacctg 4680
   aaattggaaa cttaaatgtt tctgacataa ctcccgagag cttcaatctc tcctggatgg 4740
   ctaccgatgg gatcttcgag acctttacca ttgaaattat tgattccaat aggttgctgg 4800
   agactgtgga atataatatc tctggtgctg aacgaactgc ccatatctca gggctacccc 4860
   ctagtactga ttttattgtc tacctctctg gacttgctcc cagcatccgg accaaaacca 4920
   tcagtgccac agccacgaca gaggccctgc cccttctgga aaacctaacc atttccgaca 4980
   ttaatcccta cgggttcaca gtttcctgga tggcatcgga gaatgccttt gacagctttc 5040
   tagtaacggt ggtggattct gggaagctgc tggaccccca ggaattcaca ctttcaggaa 5100
   cccagaggaa gctggagctt agaggcctca taactggcat tggctatgag gttatggtct 5160
   ctggcttcac ccaagggcat caaaccaagc ccttgagggc tgagattgtt acagaagccg 5220
   aaccggaagt tgacaacctt ctggtttcag atgccacccc agacggtttc cgtctgtcct 5280
   ggacagctga tgaaggggtc ttcgacaatt ttgttctcaa aatcagagat accaaaaagc 5340
   agtctgagcc actggaaata accctacttg cccccgaacg taccagggac ataacaggtc 5400
   tcagagaggc tactgaatac gaaattgaac tctatggaat aagcaaagga aggcgatccc 5460
   agacagtcag tgctatagca acaacagcca tgggctcccc aaaggaagtc attttctcag 5520
   acatcactga aaattcggct actgtcagct ggagggcacc cacagcccaa gtggagagct 5580
   tccggattac ctatgtgccc attacaggag gtacaccctc catggtaact gtggacggaa 5640
   ccaagactca gaccaggctg gtgaaactca tacctggcgt ggagtacctt gtcagcatca 5700
   tcgccatgaa gggctttgag gaaagtgaac ctgtctcagg gtcattcacc acagctctgg 5760
   atggcccatc tggcctggtg acagccaaca tcactgactc agaagccttg gccaggtggc 5820
   agccagccat tgccactgtg gacagttatg tcatctccta cacaggcgag aaagtgccag 5880
   aaattacacg cacggtgtcc gggaacacag tggagtatgc tctgaccgac ctcgagcctg 5940
   ccacggaata cacactgaga atctttgcag agaaagggcc ccagaagagc tcaaccatca 6000
   ctgccaagtt cacaacagac ctcgattctc caagagactt gactgctact gaggttcagt 6060
   cggaaactgc cctccttacc tggcgacccc cccgggcatc agtcaccggt tacctgctgg 6120
   tctatgaatc agtggatggc acagtcaagg aagtcattgt gggtccagat accacctcct 6180
   acagcctggc agacctgagc ccatccaccc actacacagc caagatccag gcactcaatg 6240
   ggcccctgag gagcaatatg atccagacca tcttcaccac aattggactc ctgtacccct 6300
   tccccaagga ctgctcccaa gcaatgctga atggagacac gacctctggc ctctacacca 6360
   tttatctgaa tggtgataag gctgaggcgc tggaagtctt ctgtgacatg acctctgatg 6420
   ggggtggatg gattgtgttc ctgagacgca aaaacggacg cgagaacttc taccaaaact 6480
   ggaaggcata tgctgctgga tttggggacc gcagagaaga attctggctt gggctggaca 6540
   acctgaacaa aatcacagcc caggggcagt acgagctccg ggtggacctg cgggaccatg 6600
   gggagacagc ctttgctgtc tatgacaagt tcagcgtggg agatgccaag actcgctaca 6660
   agctgaaggt ggaggggtac agtgggacag caggtgactc catggcctac cacaatggca   6720
   gatccttctc cacctttgac aaggacacag attcagccat caccaactgt gctctgtcct   6780
   acaaaggggc tttctggtac aggaactgtc accgtgtcaa cctgatgggg agatatgggg   6840
   acaataacca cagtcagggc gttaactggt tccactggaa gggccacgaa cactcaatcc   6900
   agtttgctga gatgaagctg agaccaagca acttcagaaa tcttgaaggc aggcgcaaac   6960
   gggcataaat tccagggacc actgggtgag agaggaataa ggcccagagc gaggaaagga   7020
   ttttaccaaa gcatcaatac aaccagccca accatcggtc cacacctggg catttggtga   7080
   gagtcaaagc tgaccatgga tccctggggc caacggcaac agcatgggcc tcacctcctc   7140
   tgtgatttct ttctttgcac caaagacatc agtctccaac atgtttctgt tttgttgttt   7200
   gattcagcaa aaatctccca gtgacaacat cgcaatagtt ttttacttct cttaggtggc   7260
   tctgggaatg ggagaggggt aggatgtaca ggggtagttt gttttagaac cagccgtatt   7320
   ttacatgaag ctgtataatt aattgtcatt atttttgtta gcaaagatta aatgtgtcat   7380
   tggaagccat cccttttttt acatttcata caacagaaac cagaaaagca atactgtttc   7440
   cattttaagg atatgattaa tattattaat ataataatga tgatgatgat gatgaaaact   7500
   aaggattttt caagagatct ttctttccaa aacatttctg gacagtacct gattgtattt   7560
   tttttttaaa taaaagcaca agtacttttg agtttgttaa aaaaaaaaaa aaaaaa       7616